(19) Europäisches Patentamt
European Patent Office
Office européen des brevets

(11) EP 1 504 001 B1

(12) **EUROPÄISCHE PATENTSCHRIFT**

(45) Veröffentlichungstag und Bekanntmachung des Hinweises auf die Patenterteilung:
**15.04.2009 Patentblatt 2009/16**

(21) Anmeldenummer: **03742510.5**

(22) Anmeldetag: **06.02.2003**

(51) Int Cl.:
***C07D 401/04*** *(2006.01)* ***A01N 43/54*** *(2006.01)*

(86) Internationale Anmeldenummer:
**PCT/EP2003/001162**

(87) Internationale Veröffentlichungsnummer:
**WO 2003/070721 (28.08.2003 Gazette 2003/35)**

(54) **2-(2-PYRIDYL)-5-PHENYL-6-AMINOPYRIMIDINE, VERFAHREN UND ZWISCHENPRODUKTE ZU IHRER HERSTELLUNG UND IHRE VERWENDUNG ZUR BEKÄMPFUNG VON SCHADPILZEN**

2-(2-PYRIDYL)-5-PHENYL-6-AMINOPYRIMIDINE, METHOD AND INTERMEDIATE PRODUCTS FOR THE PRODUCTION AND USE THEREOF FOR COMBATING NOXIOUS FUNGI

2-(2-PYRIDYL)-5-PHENYL-6-AMINOPYRIMIDINES, PROCEDES ET PRODUITS INTERMEDIAIRES PERMETTANT DE LES PRODUIRE ET LEUR UTILISATION POUR LUTTER CONTRE DES CHAMPIGNONS NUISIBLES

(84) Benannte Vertragsstaaten:
**AT BE BG CH CY CZ DE DK EE ES FI FR GB GR HU IE IT LI LU MC NL PT SE SI SK TR**

(30) Priorität: **21.02.2002 DE 10207428**
**08.03.2002 DE 10210136**

(43) Veröffentlichungstag der Anmeldung:
**09.02.2005 Patentblatt 2005/06**

(73) Patentinhaber: **BASF SE**
**67056 Ludwigshafen (DE)**

(72) Erfinder:
- **SCHIEWECK, Frank**
  **67258 Hessheim (DE)**
- **TORMO I BLASCO, Jordi**
  **69514 Laudenbach (DE)**
- **GROTE, Thomas**
  **67157 Wachenheim (DE)**
- **GYPSER, Andreas**
  **68159 Mannheim (DE)**
- **MÜLLER, Bernd**
  **67227 Frankenthal (DE)**
- **RHEINHEIMER, Joachim**
  **67063 Ludwigshafen (DE)**
- **ROSE, Ingo**
  **68159 Mannheim (DE)**
- **SCHÄFER, Peter**
  **67308 Ottersheim (DE)**
- **GRAMMENOS, Wassilios**
  **67071 Ludwigshafen (DE)**
- **GEWEHR, Markus**
  **56288 Kastellaun (DE)**
- **SCHWÖGLER, Anja**
  **68165 Mannheim (DE)**
- **BLETTNER, Carsten**
  **68165 Mannheimafen (DE)**
- **AMMERMANN, Eberhard**
  **64646 Heppenheim (DE)**
- **STRATHMANN, Siegfried**
  **67117 Limburgerhof (DE)**
- **LORENZ, Gisela**
  **67434 Hambach (DE)**
- **STIERL, Reinhard**
  **67251 Freinsheim (DE)**

(56) Entgegenhaltungen:
**US-A- 5 250 530**

- **H.-J. KABBE: "SUBSTITUIERTE 4-HYDROXY- UND 4-AMINO-PYRIMIDINE" LIEBIGS ANNALEN DER CHEMIE., Bd. 704, 1967, Seiten 144-9, XP002243074 VERLAG CHEMIE GMBH. WEINHEIM., DE ISSN: 0170-2041**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 504 001 B1

Printed by Jouve, 75001 PARIS (FR)

## Beschreibung

Die vorliegende Erfindung betrifft
2-(2-Pyridyl)-5-phenyl-6-aminopyrimidine der Formel I,

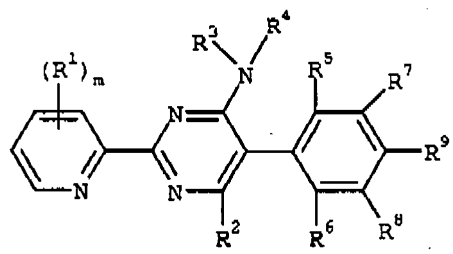

I

in der die Substituenten und der Index folgende Bedeutung haben:

$R^1$ Halogen, Hydroxy, Cyano, Oxo, Nitro, Amino, Mercapto, $C_1$-$C_6$-Alkyl, $C_1$-$C_6$-Halogenalkyl, $C_2$-$C_6$-Alkenyl, $C_2$-$C_6$-Alkinyl, $C_3$-$C_6$-Cycloalkyl, $C_1$-$C_6$-Alkoxy, $C_1$-$C_6$-Halogenalkoxy, Carboxyl, $C_1$-$C_7$-Alkoxycarbonyl, Carbamoyl, $C_1$-$C_7$-Alkylaminocarbonyl, $C_1$-$C_6$-Alkyl-$C_1$-$C_6$-alkylamincarbonyl, Morpholinocarbonyl, Pyrrolidinocarbonyl, $C_1$-$C_7$-Alkylcarbonylamino, $C_1$-$C_6$-Alkylamino, Di-($C_1$-$C_6$-alkyl)amino, $C_1$-$C_6$-Alkylthio, $C_1$-$C_6$-Alkylsulfinyl, $C_1$-$C_6$-Alkylsulfonyl, Hydroxysulfonyl, Aminosulfonyl, $C_1$-$C_6$-Alkylaminosulfonyl, Di-($C_1$-$C_6$-alkyl)aminosulfonyl;

m 0, 1, 2, 3 oder 4;

$R^2$ Halogen, Cyano, $C_1$-$C_6$-Alkyl, $C_1$-$C_6$-Halogenalkyl oder $C_1$-$C_6$-Alkoxy;

$R^3$, $R^4$ unabhängig voneinander Wasserstoff, $C_1$-$C_6$-Alkyl, $C_1$-$C_6$-Halogenalkyl, $C_3$-$C_6$-Cycloalkyl. $C_3$-$C_6$-Halogencycloalkyl, $C_2$-$C_6$-Alkenyl, $C_2$-$C_6$-Halogenalkenyl, $C_3$-$C_6$-Cycloalkenyl, $C_2$-$C_6$-Alkinyl, $C_2$-$C_6$-Halogenalkinyl oder $C_3$-$C_6$-Cycloalkinyl,
$R^3$ und $R^4$ können auch zusammen mit dem Stickstoffatom, an das siegebundensind, einen fünf-oder sechsgliedrigen Ring bilden, der durch ein Atom aus der Gruppe O, N oder S unterbrochen sein und/oder einen oder mehrere Substituenten aus der Gruppe Halogen, $C_1$-$C_6$-Alkyl, $C_1$-$C_6$-Halogenalkyl oder Oxy-$C_1$-$C_3$-alkylenoxy tragen kann oder in dem zwei benachbartes C-Atom durch eine $C_1$-$C_4$-Alkylenkette verbunden sein können;

$R^5$ Halogen, $C_1$-$C_6$-Alkyl oder $C_1$-$C_6$-Halogenalkyl;

$R^6$ Wasserstoff oder eine der bei $R^5$ genannten Gruppen;

$R^7$, $R^8$ unabhängig voneinander Wasserstoff, Halogen, $C_1$-$C_6$-Alkyl oder $C_1$-$C_6$-Halogenalkyl;

$R^9$ Wasserstoff, Halogen, Hydroxy, Cyano, $C_1$-$C_6$-Alkyl, $C_1$-$C_6$-Alkoxy, $C_3$-$C_6$-Cycloalkoxy, $C_1$-$C_6$-Halogenalkoxy, $C_1$-$C_6$-Alkoxycarbonyl oder $C_1$-$C_6$-Alkylaminocarbonyl.

Außerdem betrifft die Erfindung Verfahren und Zwischenprodukte zur Herstellung dieser Verbindungen sowie deren Verwendung zur Bekämpfung von Schadpilzen.
2-Pyridylaminopyrimidin-Derivate mit fungizider Wirkung sind aus EP-A 407 899 allgemein bekannt. Sie sind als Pflanzenschutzmittel gegen Schadpilze geeignet.
Ihre Wirkung ist jedoch in vielen Fällen nicht zufriedenstellend. Daher lag als Aufgabe zugrunde, Verbindungen mit verbesserter Wirksamkeit zu finden.
Demgemäß wurden die eingangs definierten Phenylpyrimidinderivate I gefunden. Außerdem wurden Verfahren und Zwischenprodukte zu ihrer Herstellung sowie sie enthaltende Mittel zur Bekämpfung von Schadpilzen und ihre Verwendung in diesem Sinne gefunden.
Die Verbindungen der Formel I weisen eine gegenüber den bekannten Verbindungen erhöhte Wirksamkeit gegen Schadpilze auf.
Die Verbindungen I unterscheiden sich von den bekannten Verbindungen durch die Substitution des 5-Phenylringes, in der mindestens ein Orthosubstituent nicht Wasserstoff ist.

Verbindungen der Formel I sind beispielsweise nach folgendem Syntheseweg zugänglich,

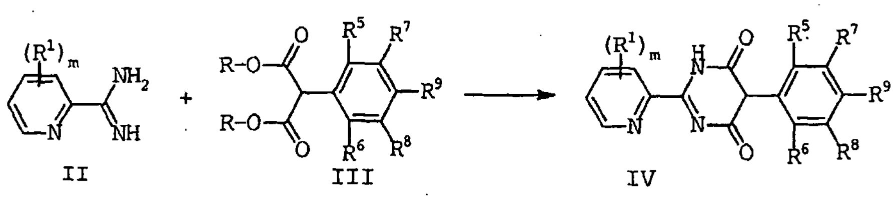

II III IV

wobei in Formel III R für $C_1$-$C_6$-Alkyl steht. Die Reaktion erfolgt üblicherweise in einem protischen Lösungsmittel wie z.B. Alkoholen, insbesondere Ethanol. Sie kann aber auch in aprotischen Lösungsmitteln wie z.B. Pyridin, N,N-Dimethylformamid, N,N-Dimethylacetamid, oder Mischungen aus diesen durchgeführt werden. Die Reaktion wird üblicherweise bei 50 bis 250°C, vorzugsweise bei 100 bis 200°C durchgeführt [vgl.: Austr. J. Chem., Bd. 32, S. 669-679 (1979); J. Org. Chem., Bd. 58, S. 3785-3786 (1993); Arm. Xim. ZH, Bd. 38, N11, 718-719 (1985)].

In der Regel ist es vorteilhaft, in Gegenwart einer Base zu arbeiten, die äquimolar oder auch in Überschuß angewendet werden kann. Als Basen kommen Alkalimetall- und Erdalkalimetallalkoholate wie Natriummethanolat, Natriumethanolat, Kaliumethanolat, Kalium- tert.-Butanolat, insbesondere Natriumethylat oder auch Stickstoffbasen, wie Triethylamin, Tri-isopropylethylamin und N-Methylpiperidin, insbesondere Pyridin und Tributylamin in Frage.

Üblicherweise werden die Komponenten in etwa stöchiometrischen Mengen eingesetzt. Es kann jedoch auch vorteilhaft sein eine der Komponenten im Überschuß einzusetzen.

Die Ausgangsstoffe der Formeln II und III sind in der Literatur bekannt [vgl. EP-A 588 146; EP-A 10 02 788; WO-A 98/41496], z. T. auch kommerziell erhältlich oder können gemäß der zitierten Literatur hergestellt werden.

Verbindungen IV werden in die Dichlorpyrimidine der Formel V überführt [vgl. US 4,963,678; EP-A 745 593; DE-A 196 42 533; WO-A 99/32458; J.Org. Chem. Bd. 58, S. 3785-3786 (1993); Helv. Chim. Acta, Bd. 64, S. 113-152 (1981)].

IV

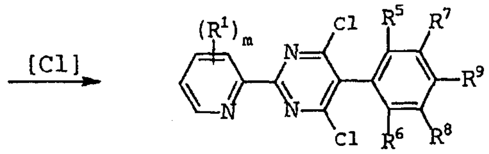


V

Als Chlorierungsmittel [C1] eignen sich beispielsweise $POCl_3$, $PCl_3/Cl_2$ oder $PCl_5$, oder Mischungen dieser Reagenzien. Die Reaktion kann in überschüssigem Chlorierungsmittel ($POCl_3$) oder einem inerten Lösungsmittel, wie beispielsweise Acetonitril oder 1,2-Dichlorethan durchgeführt werden. Die Durchführung in $POCl_3$ ist bevorzugt.

Diese Umsetzung erfolgt üblicherweise zwischen 10 und 180°C. Aus praktischen Gründen entspricht gewöhnlich die Reaktionstemperatur der Siedetemperatur des eingesetzten Chlorierungsmittels ($POCl_3$) oder des Lösungsmittels. Das Verfahren wird vorteilhaft unter Zusatz von N,N-Dimethylformamid in katalytischen oder unterstöchiometrischen Mengen oder von Stickstoffbasen, wie beispielsweise N,N-Dimethylaminilin durchgeführt.

V +

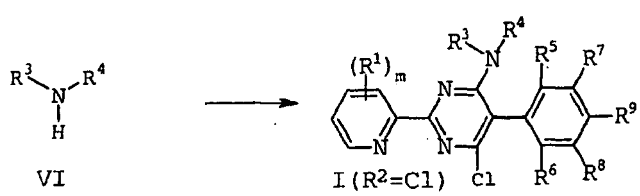

VI I($R^2$=Cl)

Durch Aminierung mit VI werden die Dichlorverbindungen der Formel V in die Verbindungen der Formel I, in der $R^2$ für Chlor steht, überführt.

Diese Umsetzung erfolgt üblicherweise bei 0 bis 150°C, vorzugsweise bei 20 bis 120°C [vgl. J. Chem. Res. S (7), S. 286-287 (1995), Liebigs Ann. Chem., S. 1703-1705 (1995)] in einem inerten Lösungsmittel gegebenenfalls in Gegenwart einer Hilfsbase.

Als Lösungsmittel kommen protische Lösungsmittel, wie Alkohole, beispielsweise Ethanol, oder aprotische Lösungsmittel, wie aromatische Kohlenwasserstoffe oder Ether, beispielsweise Toluol, o-, m- und p-Xylol, Diethylether,

Diisopropylether, tert.-Butylmethylether, Dioxan oder Tetrahydrofuran, insbesondere tert. Butylmethylether oder Tetrahydrofuran, in Frage. Als Hilfsbase kommen beispielsweise die im folgenden genannten in Betracht: $NaHCO_3$, $Na_2CO_3$, $Na_2HPO_4$, $Na_2B_4O_7$, Diethylanilin oder Ethyldiisopropylamin.

Üblicherweise werden die Komponenten in etwa stöchiometrischem Verhältnis eingesetzt. Es kann jedoch vorteilhaft sein, das Amin im Überschuß einzusetzen.

Die Amine der Formel VI sind käuflich oder literaturbekannt oder können nach bekannten Methoden hergestellt werden.

Verbindungen der Formel I, in der $R^2$ für Alkoxy steht, werden aus den entsprechenden Chlorverbindungen der Formel I, in der $R^2$ für Chlor steht, durch Umsetzung mit Alkali- oder Erdalkalimetallalkoholaten erhalten [vgl.: Heterocycles, Bd. 32, S. 1327-1340 (1991); J. Heterocycl. Chem. Bd. 19, S. 1565-1567 (1982); Geterotsikl. Soedin, S. 400-402 (1991)].

Verbindungen der Formel I, in der $R^2$ für Cyano steht, werden aus den entsprechenden Chlorverbindungen der Formel I ($R^2$ = Cl) durch Umsetzung mit Alkali-, Erdalkalimetall- oder Metallcyaniden, wie NaCN, KCN oder $Zn(CN)_2$, erhalten [vgl.: Heterocycles, Bd. 39, S. 345-356 (1994); Collect. Czech. Chem. Commun. Bd. 60, S. 1386-1389 (1995) ; Acta Chim. Scand., Bd. 50, S. 58-63 (1996)].

Verbindungen der Formel I, in der $R^2$ für $C_1$-$C_6$-Alkyl oder $C_1$-$C_6$-Halogenalkyl steht (Formel Ia), lassen sich durch entsprechende Abwandlung der Ausgangsmaterialien der Formel III analog der beschriebenen Synthesefolge zu den Verbindungen I, in der $R^2$ Chlor bedeutet, herstellen. Anstatt der Phenylmalonester der Formel III werden Phenyl-β-ketoester der Formel VII, in der $R^2$ Alkyl oder Halogenalkyl bedeutet, mit dem Amidin der Formel II eingesetzt. Die folgenden Umsetzungen werden analog der vorstehend beschriebenen Synthesen zu den Verbindungen mit $R^2$ = Chlor durchgeführt.

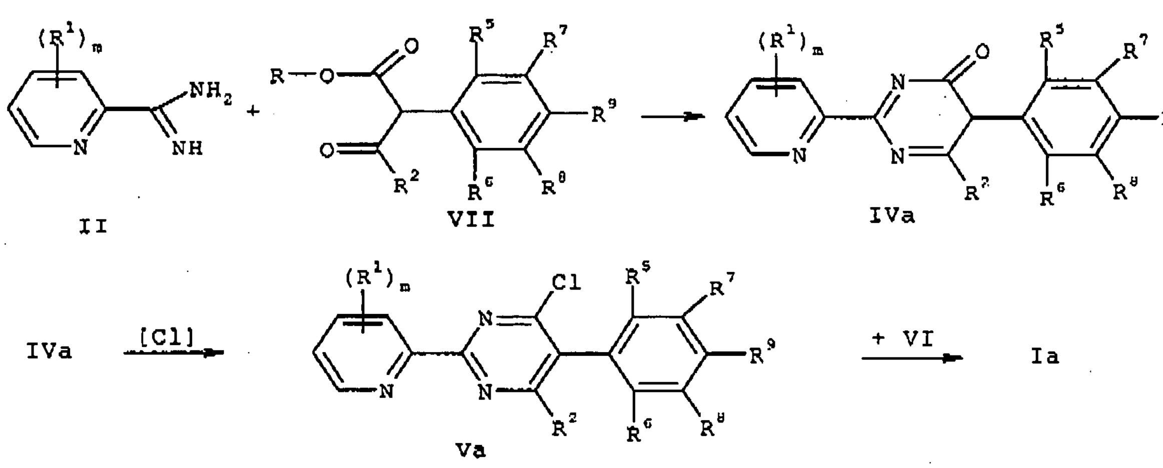


II VII IVa

IVa Va Ia

Die Verbindungen der Formeln IVa und Va sind neu.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Bei den in den vorstehenden Formeln angegebenen Definitionen der Symbole wurden Sammelbegriffe verwendet, die allgemein repräsentativ für die folgenden Substituenten stehen:

**Halogen:** Fluor, Chlor, Brom und Jod;

**Alkyl:** gesättigte, geradkettige oder verzweigte Kohlenwasserstoffreste mit 1 bis 4, 6 oder 8 Kohlenstoffatomen, z.B. $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. $C_1$-$C_2$-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 1,1,1-Trifluorprop-2-yl;

**Alkenyl:** ungesättigte, geradkettige oder verzweigte Kohlenwasserstoffreste mit 2 bis 4, 6 oder 8 Kohlenstoffatomen und einer Doppelbindung in einer beliebigen Position, z.B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;

**Alkinyl:** geradkettige oder verzweigte Kohlenwasserstoffgruppen mit 2 bis 4, 6 oder 8 Kohlenstoffatomen und einer Dreifachbindung in einer beliebigen Position, z.B. $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;

**Cycloalkyl:** monocyclische, gesättigte Kohlenwasserstoffgruppen mit 3 bis 6 Kohlenstoffringgliedern, wie Cyclopropyl, Cyclobutyl, Cyclopentyl und Cyclohexyl;

**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;

**Oxyalkylenoxy:** divalente unverzweigte Ketten aus 1 bis 3 $CH_2$-Gruppen, wobei beide Valenzen über ein Sauerstoffatom an das Gerüst gebunden ist, z.B. $OCH_2O$, $OCH_2CH_2O$ und $OCH_2CH_2CH_2O$;

Die besonders bevorzugten Ausführungsformen der Zwischenprodukte in Bezug auf die Variablen entsprechen denen der Reste $R^1$ bis $R^9$ der Formel I.

Im Hinblick auf ihre bestimmungsgemäße Verwendung der 2-Pyridylpyrimidine der Formel I sind die folgenden Bedeutungen der Substituenten, und zwar jeweils für sich allein oder in Kombination, besonders bevorzugt:

Verbindungen I werden besonders bevorzugt, in denen m = 0 ist.

Daneben werden Verbindungen I bevorzugt, in denen m = 1 oder 2 ist und $R^1$ folgende Bedeutung hat:

Halogen, Hydroxy, Cyano, Nitro, Amino, Mercapto, $C_1$-$C_6$-Alkyl, $C_1$-$C_6$-Halogenalkyl, $C_2$-$C_6$-Alkenyl, $C_2$-$C_6$-Alkinyl, $C_3$-$C_6$-Cycloalkyl, $C_1$-$C_6$-Alkoxy, $C_1$-$C_6$-Halogenalkoxy, Carboxyl, $C_1$-$C_7$-Alkoxycarbonyl, Carbamoyl, $C_1$-$C_7$-Alkylaminocarbonyl, $C_1$-$C_6$-Alkyl-$C_1$-$C_6$-alkylamincarbonyl, Morpholinocarbonyl, Pyrrolidinocarbonyl, $C_1$-$C_7$-Alkylcarbonylamino, $C_1$-$C_6$-Alkylamino, Di-($C_1$-$C_6$-alkyl)amino, $C_1$-$C_6$-Alkylthio, $C_1$-$C_6$-Alkylsulfinyl, $C_1$-$C_6$-Alkylsulfonyl, Hydroxysulfonyl, Aminosulfonyl, $C_1$-$C_6$-Alkylaminosulfonyl oder Di-($C_1$-$C_6$-alkyl)aminosulfonyl.

Insbesondere werden Verbindungen I bevorzugt, in denen $R^1$ ausgewählt ist aus der Gruppe:

Halogen, Cyano, Nitro, Amino, $C_1$-$C_6$-Alkyl, $C_1$-$C_6$-Halogenalkyl, $C_1$-$C_6$-Alkoxy,Carboxyl, $C_1$-$C_7$-Alkoxycarbonyl, Carbamoyl, $C_1$-$C_7$-Alkylaminocarbonyl, Di-($C_1$-$C_6$-alkyl)amincarbonyl oder $C_1$-$C_7$-Alkylcarbonylamino.

Bevorzugt werden weiterhin Verbindungen I, in denen m 1 ist und $R^1$ für Halogen, Cyano, Nitro, Methyl oder Methoxy steht.

Insbesondere sind Verbindungen I bevorzugt, in denen $R^1$ Fluor, Chlor oder Methyl bedeutet.

Verbindungen I werden bevorzugt, in denen $R^2$ für Halogen, $C_1$-$C_6$-Alkyl oder $C_1$-$C_6$-Alkoxy, insbesondere für Halogen, steht.

Besonders bevorzugt sind Verbindungen der Formel I, in der $R^2$ Chlor bedeutet.

Außerdem werden Verbindungen der Formel I bevorzugt, in denen $R^3$ für Wasserstoff steht.

Gleichermaßen besonders bevorzugt sind Verbindungen I, in denen $R^3$ und $R^4$ unabhängig voneinander $C_1$-$C_6$-Alkyl, $C_1$-$C_6$-Halogenalkyl, $C_3$-$C_6$-Cycloalkyl, $C_2$-$C_6$-Alkenyl bedeuten.

Insbesondere werden Verbindungen der Formel I bevorzugt, in denen $R^3$ für Wasserstoff und $R^4$ für $C_1$-$C_4$-Halogenalkyl steht.

Weiterhin werden Verbindungen I bevorzugt, in denen $R^3$ und $R^4$ zusammen mit dem Stickstoffatom, an das sie gebunden sind, einen fünf- oder sechsgliedrigen Ring bilden, der durch ein Heteroatom unterbrochen sein kann und einen oder zwei $C_1$-$C_6$-Alkylsubstituenten tragen kann.

Gleichermaßen besonders bevorzugt werden Verbindungen I, in denen $R^5$ Halogen oder Methyl und $R^6$ Wasserstoff bedeuten.

Besonders bevorzugt werden Verbindungen I, in denen $R^6$ Wasserstoff bedeutet.

Desweiteren werden Verbindungen I besonders bevorzugt, in denen $R^7$ und $R^8$ gleich oder verschieden sind und Wasserstoff oder Halogen bedeuten.

Außerdem werden Verbindungen I besonders bevorzugt, in denen $R^9$ für Wasserstoff, Halogen, $C_1$-$C_4$-Alkyl, $C_1$-$C_4$-Alkoxy, $C_1$-$C_6$-Alkoxycarbonyl oder $C_1$-$C_6$-Alkylaminocarbonyl steht.

Gleichermaßen besonders bevorzugt sind Verbindungen I', in denen $R^1$ bis $R^4$ wie für Formel I definiert sind und $R^A$ für folgende Restekombinationen steht: 2-Methyl,4-Fluor, 2-Fluor,4-Methyl, 2,4-Dimethyl, 2-Chlor,6-Fluor; 2,6-Difluor; 2,6-Dichlor; 2-Methyl,6-Fluor; 2,4,6-Trifluor; 2,6-Difluor, 4-Methoxy und Pentafluor.

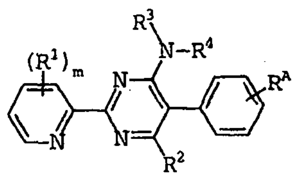

I'

Darüberhinaus werden Verbindungen der Formel I' besonders bevorzugt, in denen $R^A$ 2,4,6-Trifluor bedeutet.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

Tabelle 1

Verbindungen der Formel I-1, in denen $R^5$ für Fluor, $R^6$ für Chlor und $R^7$, $R^8$ und $R^9$ für Wasserstoff stehen und die Kombination der Reste $R^3$ und $R^4$ für eine Verbindung jeweils einer Zeile der Tabelle A entspricht

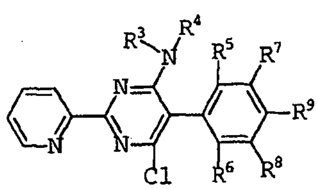

I-1

Tabelle 2

Verbindungen der Formel I-1, in denen $R^5$ und $R^6$ für Fluor und $R^7$, $R^8$ und $R^9$ für Wasserstoff stehen und die Kombination der Reste $R^3$ und $R^4$ für eine Verbindung jeweils einer Zeile der Tabelle A entspricht

Tabelle 3

Verbindungen der Formel I-1, in denen $R^5$ und $R^6$ für Chlor und $R^7$, $R^8$ und $R^9$ für Wasserstoff stehen und die Kombination der Reste $R^3$ und $R^4$ für eine Verbindung jeweils einer Zeile der Tabelle A entspricht

Tabelle 4

Verbindungen der Formel I-1, in denen $R^5$ für Fluor und $R^6$ für Methyl und $R^7$, $R^8$ und $R^9$ für Wasserstoff stehen und die Kombination der Reste $R^3$ und $R^4$ für eine Verbindung jeweils einer Zeile der Tabelle A entspricht

Tabelle 5

Verbindungen der Formel I-1, in denen $R^5$, $R^6$ und $R^9$ für Fluor und $R^7$ und $R^8$ für Wasserstoff stehen und die Kombination der Reste $R^3$ und $R^4$ für eine Verbindung jeweils einer Zeile der Tabelle A entspricht

Tabelle 6

Verbindungen der Formel I-1, in denen $R^5$ und $R^6$ für Fluor, $R^7$ und $R^8$ für Wasserstoff und $R^9$ für Methoxy stehen und die Kombination der Reste $R^3$ und $R^4$ für eine Verbindung jeweils einer Zeile der Tabelle A entspricht

Tabelle 7

Verbindungen der Formel I-1, in denen $R^5$, $R^6$, $R^7$, $R^8$ und $R^9$ für Fluor stehen und die Kombination der Reste $R^3$ und $R^4$ für eine Verbindung jeweils einer Zeile der Tabelle A entspricht

Tabelle 8

Verbindungen der Formel I-1, in denen $R^5$ für Methyl, $R^6$, $R^7$ und $R^8$ für Wasserstoff und $R^9$ für Fluor stehen und die Kombination der Reste $R^3$ und $R^4$ für eine Verbindung jeweils einer Zeile der Tabelle A entspricht

Tabelle 9

Verbindungen der Formel I-1, in denen $R^5$ für Fluor, $R^6$, $R^7$ und $R^8$ für Wasserstoff und $R^9$ für Methyl stehen und die Kombination der Reste $R^3$ und $R^4$ für eine Verbindung jeweils einer Zeile der Tabelle A entspricht

Tabelle 10

Verbindungen der Formel I-1, in denen $R^5$ und $R^9$ für Methyl und $R^6$, $R^7$ und $R^8$ für Wasserstoff stehen und die Kombination der Reste $R^3$ und $R^4$ für eine Verbindung jeweils einer Zeile der Tabelle A entspricht

Tabelle 11

Verbindungen der Formel I-1, in denen $R^5$ und $R^6$ für Fluor, $R^7$ und $R^8$ für Wasserstoff, $R^9$ für Hydroxy stehen und die Kombination der Reste $R^3$ und $R^4$ für eine Verbindung jeweils einer Zeile der Tabelle A entspricht

Tabelle A

| No. | $R^3$ | $R^4$ |
|---|---|---|
| A-1 | $CH_2CH_3$ | H |
| A-2 | $CH_2CH_3$ | $CH_3$ |
| A-3 | $CH_2CH_3$ | $CH_2CH_3$ |
| A-4 | $CH_2CH_2CH_3$ | H |
| A-5 | $CH_2CH_2CH_3$ | $CH_3$ |
| A-6 | $CH_2CH_2CH_3$ | $CH_2CH_3$ |
| A-7 | $CH_2CH_2CH_3$ | $CH_2CH_2CH_3$ |

(fortgesetzt)

| No. | $R^3$ | $R^4$ |
|---|---|---|
| A-8 | $CH_2$-$CH_2F$ | H |
| A-9 | $CH_2$-$CH_2F$ | $CH_3$ |
| A-10 | $CH_2$-$CH_2F$ | $CH_2CH_3$ |
| A-11 | $CH_2CF_3$ | H |
| A-12 | $CH_2CF_3$ | $CH_3$ |
| A-13 | $CH_2CF_3$ | $CH_2CH_3$ |
| A-14 | $CH_2CF_3$ | $CH_2CH_2CH_3$ |
| A-15 | $CH_2CCl_3$ | H |
| A-16 | $CH_2CCl_3$ | $CH_3$ |
| A-17 | $CH_2CCl_3$ | $CH_2CH_3$ |
| A-18 | $CH_2CCl_3$ | $CH_2CH_2CH_3$ |
| A-19 | $CH(CH_3)_2$ | H |
| A-20 | $CH(CH_3)_2$ | $CH_3$ |
| A-21 | $CH(CH_3)_2$ | $CH_2CH_3$ |
| A-22 | $CH(CH_3)_2$ | $CH_2CH_2CH_3$ |
| A-23 | $CH_2C(CH_3)_3$ | H |
| A-24 | $CH_2C(CH_3)_3$ | $CH_3$ |
| A-25 | $CH_2C(CH_3)_3$ | $CH_2CH_3$ |
| A-26 | $CH_2CH(CH_3)_2$ | H |
| A-27 | $CH_2CH(CH_3)_2$ | $CH_3$ |
| A-28 | $CH_2CH(CH_3)_2$ | $CH_2CH_3$ |
| A-29 | (±) $CH(CH_2CH_3)CH_3$ | H |
| A-30 | (±) $CH(CH_2CH_3)CH_3$ | $CH_3$ |
| A-31 | (±) $CH(CH_2CH_3)CH_3$ | $CH_2CH_3$ |
| A-32 | (R) $CH(CH_2CH_3)CH_3$ | H |
| A-33 | (R) $CH(CH_2CH_3)CH_3$ | $CH_3$ |
| A-34 | (R) $CH(CH_2CH_3)CH_3$ | $CH_2CH_3$ |
| A-35 | (S) $CH(CH_2CH_3)CH_3$ | H |
| A-36 | (S) $CH(CH_2CH_3)CH_3$ | $CH_3$ |
| A-37 | (S) $CH(CH_2CH_3)CH_3$ | $CH_2CH_3$ |
| A-38 | (±) $CH(CH_3)$-$CH(CH_3)_2$ | H |
| A-39 | (±) $CH(CH_3)$-$CH(CH_3)_2$ | $CH_3$ |
| A-40 | (±) $CH(CH_3)$-$CH(CH_3)_2$ | $CH_2CH_3$ |
| A-41 | (R) $CH(CH_3)$-$CH(CH_3)_2$ | H |
| A-42 | (R) $CH(CH_3)$-$CH(CH_3)_2$ | $CH_3$ |
| A-43 | (R) $CH(CH_3)$-$CH(CH_3)_2$ | $CH_2CH_3$ |
| A-44 | (S) $CH(CH_3)$-$CH(CH_3)_2$ | H |
| A-45 | (S) $CH(CH_3)$-$CH(CH_3)_2$ | $CH_3$ |

(fortgesetzt)

| No. | $R^3$ | $R^4$ |
|---|---|---|
| A-46 | (S) $CH(CH_3)$-$CH(CH_3)_2$ | $CH_2CH_3$ |
| A-47 | (±) $CH(CH_3)$-$C(CH_3)_3$ | H |
| A-48 | (±) $CH(CH_3)$-$C(CH_3)_3$ | $CH_3$ |
| A-49 | (±) $CH(CH_3)$-$C(CH_3)_3$ | $CH_2CH_3$ |
| A-50 | (R) $CH(CH_3)$-$C(CH_3)_3$ | H |
| A-51 | (R) $CH(CH_3)$-$C(CH_3)_3$ | $CH_3$ |
| A-52 | (R) $CH(CH_3)$-$C(CH_3)_3$ | $CH_2CH_3$ |
| A-53 | (S) $CH(CH_3)$-$C(CH_3)_3$ | H |
| A-54 | (S) $CH(CH_3)$-$C(CH_3)_3$ | $CH_3$ |
| A-55 | (S) $CH(CH_3)$-$C(CH_3)_3$ | $CH_2CH_3$ |
| A-56 | (±) $CH(CH_3)$-$CF_3$ | H |
| A-57 | (±) $CH(CH_3)$-$CF_3$ | $CH_3$ |
| A-58 | (±) $CH(CH_3)$-$CF_3$ | $CH_2CH_3$ |
| A-59 | (R) $CH(CH_3)$-$CF_3$ | H |
| A-60 | (R) $CH(CH_3)$-$CF_3$ | $CH_3$ |
| A-61 | (R) $CH(CH_3)$-$CF_3$ | $CH_2CH_3$ |
| A-62 | (S) $CH(CH_3)$-$CF_3$ | H |
| A-63 | (S) $CH(CH_3)$-$CF_3$ | $CH_3$ |
| A-64 | (S) $CH(CH_3)$-$CF_3$ | $CH_2CH_3$ |
| A-65 | (±) $CH(CH_3)$-$CCl_3$ | H |
| A-66 | (±) $CH(CH_3)$-$CCl_3$ | $CH_3$ |
| A-67 | (±) $CH(CH_3)$-$CCl_3$ | $CH_2CH_3$ |
| A-68 | (R) $CH(CH_3)$-$CCl_3$ | H |
| A-69 | (R) $CH(CH_3)$-$CCl_3$ | $CH_3$ |
| A-70 | (R) $CH(CH_3)$-$CCl_3$ | $CH_2CH_3$ |
| A-71 | (S) $CH(CH_3)$-$CCl_3$ | H |
| A-72 | (S) $CH(CH_3)$-$CCl_3$ | $CH_3$ |
| A-73 | (S) $CH(CH_3)$-$CCl_3$ | $CH_2CH_3$ |
| A-74 | $CH_2C(CH_3)=CH_2$ | H |
| A-75 | $CH_2C(CH_3)=CH_2$ | $CH_3$ |
| A-76 | $CH_2C(CH_3)=CH_2$ | $CH_2CH_3$ |
| A-77 | Cyclopentyl | H |
| A-78 | Cyclopentyl | $CH_3$ |
| A-79 | Cyclopentyl | $CH_2CH_3$ |
| A-80 | $-(CH_2)_4-$ | |
| A-81 | (±) $-(CH_2)_2$-$CH(CH_3)$-$CH_2-$ | |
| A-82 | (R) $-(CH_2)_2$-$CH(CH_3)$-$CH_2-$ | |
| A-83 | (S) $-(CH_2)_2$-$CH(CH_3)$-$CH_2-$ | |

(fortgesetzt)

| No. | $R^3$ | $R^4$ |
|---|---|---|
| A-84 | $-(CH_2)_2-CH(OCH_3)-CH_2-$ | |
| A-85 | $-(CH_2)_2-CH(CH_2CH_3)-CH_2-$ | |
| A-86 | $-(CH_2)_2-CH[CH(CH_3)_2]-CH_2-$ | |
| A-87 | $-CH_2-CH=CH-CH_2-$ | |
| A-88 | $-(CH_2)_5-$ | |
| A-89 | $-(CH_2)_2-CH(CH_3)-(CH_2)_2-$ | |
| A-90 | $(\pm)$ $-(CH_2)_3-CH(CH_3)-CH_2-$ | |
| A-91 | (R) $-(CH_2)_3-CH(CH_3)-CH_2-$ | |
| A-92 | (S)$-(CH_2)_3-CH(CH_3)-CH_2-$ | |
| A-93 | $-(CH_2)_2-C(O[CH_2]_2O)-(CH_2)_2-$ | |
| A-94 | $-(CH_2)_2-C(O[CH_2]_3O)-(CH_2)_2-$ | |
| A-95 | 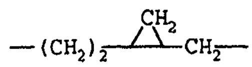 | |
| A-96 | $-(CH_2)_2-CH=CH-CH_2-$ | |
| A-97 | $-(CH_2)_2-O-(CH_2)_2-$ | |
| A-98 | $-CH_2-CH(CH_3)-O-CH(CH_3)-CH_2-$ | |
| A-99 | (cis) $-CH_2-CH(CH_3)-O-CH(CH_3)-CH_2-$ | |
| A-100 | (trans) $-CH_2-CH(CH_3)-O-CH(CH_3)-CH_2-$ | |
| A-101 | $-(CH_2)_2-NH-(CH_2)_2-$ | |
| A-102 | $-(CH_2)_2-N(CH_3)-(CH_2)_2-$ | |
| A-103 | $-(CH_2)_2-S-(CH_2)_2-$ | |
| A-104 | $-(CH_2)_2-CHF-(CH_2)_2-$ | |
| A-105 | $-(CH_2)_3-CHF-CH_2-$ | |
| A-106 | $-(CH_2)_2-CH(CF_3)-(CH_2)_2-$ | |
| A-107 | $-(CH_2)_2-CH(CH_2F)-(CH_2)_2-$ | |
| A-108 | $-(CH_2)_2-CF_2-(CH_2)_2-$ | |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:

- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Blumeria graminis (echter Mehltau) an Getreide,
- Fusarium- und Verticillium-Arten an verschiedenen Pflanzen,

- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Aminoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:

I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.

II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).

III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).

IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).

V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).

VI. Man vermischt 90 Gew.-Teile einer erfindungsgemäßen Verbindung mit 10 Gew.-Teilen N-Methyl-$\alpha$-pyrrolidon und erhält eine Lösung, die zur Anwendung in Form kleinster Tropfen geeignet ist (Wirkstoffgehalt 90 Gew.-%).

VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

VIII. 20 Gew.-Teile einer erfindungsgemäßen Verbindung werden mit 3 Gew.-Teilen des Natriumsalzes der Diisobutylnaphthalin-$\alpha$sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier-

oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.
Die folgende Liste von Fungiziden, mit denen die erfindungsgemäßen Verbindungen gemeinsam angewendet werden können, soll die Kombinationsmöglichkeiten erläutern, nicht aber einschränken:

- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester,2-Methoxycarbonylamino-benzimidazol,2-(Fury1-(2))-benzimidazol,2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-y1-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yl-oxy]-phenyl}-3-methoxyacrylat, Methyl-E-methoxyimino-[a-(2-phenoxyphenyl)]-acetamid, Methyl-E-methoxyimino-[α-(2,5-dimethylphenoxy)-o-tolyl]-acetamid, Methyl-E-2-{2-[2-trifluormethylpyridyl-6-]oxymethyl]-phenyl}3-methoxyarrylat,(E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)N-methoxy-carbamat,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-a-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol, Iprovalicarb, Benthiavalicarb, Proquinazid, 5-Chlor-2-cyano-4-p-tolyl-imidazol-1-sulfonsäuredimethylamid, 3,5-Dichlor-N-(3-chlor-1-ethyl-1-methyl-2-oxo-propyl)-4-methyl-benzamid.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

Beispiel 1: 6-Chlor-5-(2,4,6-trifluorphenyl)-4-isopropylamino-2-(2-pyridyl)-pyrimidin [I-1]

a) 4,6-Dihydroxy-5-(2,4,6-trifluorphenyl)-2-(2-pyridyl)-pyrimidin

332 g (1,16 mol) 2-(2,4,6-Trifluorphenyl)malonsäurediethylester und 192 g (1,04 mol) Tributylamin wurden zusammen mit 182 g (1,17 mol) Pyridin-2-carboxamidin für acht Std. auf 180°C erhitzt. Dabei destillierte das entstehende Ethanol ab. Anschließend lies man auf 60-70°C abkühlen und versetzte mit einer Lösung von 116 g (2,89 mol) Natriumhydroxid in 1200 ml Wasser. Nach 30 min. Rühren und Abkühlen auf etwa 20-25°C wurde mit Methyl-tert-butylether (MTBE) extrahiert und nach Phasentrennung die Wasserphase mit 6N Salzsäure angesäuert. Nach Abfiltrieren und Trocknen wurden 180 g der Titelverbindung erhalten.
$^1$H-NMR: δ (ppm, DMSO-$d_6$) = 8,75 (d); 8,3 (d); 8,1 (t); 7,6 (m); 7,1 (t).

b) 4,6-Dichlor-5-(2,4,6-trifluorphenyl)-2-(2-pyridyl)-pyrimidin

Ein Suspension von 80,4 g (0,252 mol) des Pyrimidins aus Bsp. 1a in 515 g (3,36 mol) Phosphoroxychlorid wurde für 8 Std. auf 120°C erhitzt, dann im Vakuum einengt. Der Rückstand wurde in Dichlormethan und Wasser aufgenommen. Nach Phasentrennung wurde die organische Phase getrocknet und vom Lösungsmittel befreit. Nach Chomatographie an Kieselgel (Cyclohexan/Essigester) wurden 26 g der Tielverbindung erhalten.
$^1$H-NMR: δ (ppm, $CDCl_3$) = 8,9 (d); 8,6 (d); 7,8 (t); 7,5 (m); 6,8 (t).

c) 6-Chlor-5-(2,4,6-trifluorphenyl)-4-isopropylamino-2-(2-pyridyl)-pyrimidin

Eine Lösung von 3,30 g (0,093 mol) des Dichlorids aus Bsp. 1b in 4,5 ml Dimethylformamid (DMF) und 2,7 g (0,046 mol) Isopropylamin wurde 24 Std. bei 40°C gerührt. Nach Abkühlen auf 20-25°C wurde die Mischung mit Dichlormethan extrahiert, die organische Phase mit Wasser und ges. $NaHCO_3$-Lösung gewaschen, dann getrocknet und vom Lösungsmittel befreit. Nach Chomatographie an Kieselgel (Cyclohexan/Essigester) wurden 3,05 g der Tielverbindung erhalten.
$^1$H-NMR: δ (ppm, $CDCl_3$) = 8,8 (d); 8,4 (d); 7,8 (t); 7,4 (dd); 6,8 (t); 4,5 (m); 4,4 (m); 1,4 (s); 1,25 (d).

Tabelle I

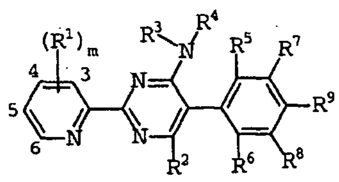

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[˚C], $^1$H-NMR[ppm]; $logP_{ow}$ |
|---|---|---|---|---|---|---|---|---|---|---|
| I-1 | H | Cl | $CH(CH_3)_2$ | H | F | F | H | H | F | (Bsp. 1) |
| I-2 | H | Cl | $CH_2C(=CH_2)CH_3$ | $CH_2CH_3$ | Cl | F | H | H | H | $logP_{ow}$ 4,73 |
| I-3 | H | Cl | $CH(CH_3)_2$ | H | Cl | F | H | H | H | $logP_{ow}$ 3,59 |
| I-4 | H | Cl | $c-C_5H_9$ | H | Cl | F | H | H | H | 152-156 |
| I-5 | H | Cl | $CH_2CH_3$ | $CH_2CH_3$ | Cl | F | H | H | H | $logP_{ow}$ 4,23 |
| I-6 | H | Cl | (S) $CH(CH_3)C(CH_3)_3$ | H | Cl | F | H | H | H | 144-146 |
| I-7 | H | Cl | (R) $CH(CH_3)C(CH_3)_3$ | H | Cl | F | H | H | H | $logP_{ow}$ 4,8 |
| I-8 | H | Cl | $-(CH_2)_2-CH(CH_3)-(CH_2)_2-$ | | Cl | F | H | H | H | 0,9 (d,3H); 1,1 (m,2H); 1,6 (m,3H); 2,9 (m,2H); 4,1 (m,2H); 7,1 (t,1H); 7,4 (m,2H); 7,8 (t, 1H); 8,4 (d, 1H); 8,9 (m, 1H) |
| I-9 | H | Cl | $CH_2CH_3$ | $CH_2CH_3$ | F | F | H | H | F | $logP_{ow}$ 4,08 |
| I-10 | H | Cl | (S) $CH(CH_3)C(CH_3)_3$ | H | F | F | H | H | F | $logP_{ow}$ 4,75 |
| I-11 | H | Cl | $c-C_5H_9$ | H | F | F | H | H | F | 1,4-1,7 (m, 4H), 1,8 (m, 2H); 2,1 (m, 2H); 4,6 (m, 2H); 6,9 (dd, 2H); 7,4 (m, 1H); 7,8 (t, 1H); 8,5 (d, 1H); 8,8 (d, 1H) |
| I-12 | H | Cl | (S) $CH(CH_3)CF_3$ | H | F | F | H | H | F | $logP_{ow}$ 4,5 |
| I-13 | H | Cl | (S) $CH(CH_3)CH(CH_3)_2$ | H | F | F | H | H | F | 187 |
| I-14 | H | Cl | $CH_2C(CH_3)_3$ | H | F | F | H | H | F | 162 |
| I-15 | H | Cl | (R) $CH(CH_3)C(CH_3)_3$ | H | F | F | H | H | F | 139 |
| I-16 | H | Cl | (R) $CH(CH_3)CH(CH_3)_2$ | H | F | F | H | H | F | 185 |
| I-17 | $6-CH_3$ | Cl | $CH(CH_3)_2$ | H | F | F | H | H | F | 234-236 |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-18 | 6-$CH_3$ | Cl | (S) $CH(CH_3)CF_3$ | H | F | F | H | H | F | 238 |
| I-19 | 6-$CH_3$ | Cl | $CH_2CH_3$ | $CH_2CH_3$ | F | F | H | H | F | 1,0 (t,6H); 2,7 (s,3H); 3,4 (q, 4H); 6,8 (m, 2H); 7,3 (d, 1H); 7,7 (t, 1H); 8,2 (d, 1H) |
| I-20 | 6-$CH_3$ | Cl | $CH_2C(CH_3)_3$ | H | F | F | H | H | F | 184 |
| I-21 | 6-$CH_3$ | Cl | $CH(CH_3)_2$ | $CH_3$ | F | F | H | H | F | 1,1 (d,6H); 2,55 (s,3H); 2,7 (s,3H); 4,8 (sept., 1H); 6,8 (dd, 2H); 7,3 (m, 1H); 7,7 (t, 1H); 8,2 (d, 1H) |
| I-22 | 6-$CH_3$ | Cl | $CH_2CH(CH_3)_2$ | $CH_2CH_3$ | F | F | H | H | F | 0,8 (d,6H); 1,0 (t,3H); 2,0 (sept.,1H), 2,7 (s,3H); 3,2 (d,2H); 3,3 (q,2H); 6,8 (dd,2H); 7,3 (d,1H); 7,7 (t,1H); 8,13 (d,1H) |
| I-23 | 6-$CH_3$ | Cl | $CH_2C(=CH_2)CH_3$ | $CH_2CH_3$ | F | F | H | H | F | 1,1 (t,3H); 1,45 (s,3H); 2,7 (s,3H); 3,4 (q,2H); 3,9 (s,2H); 4,8 (s,2H); 6,75 (dd,2H); 7,3 (m,1H); 7,7 (t,1H); 8,2 (d,1H) |
| I-24 | 6-$CH_3$ | Cl | $-(CH_2)_2-O-(CH_2)_2-$ | | F | F | H | H | F | $logP_{ow}$ 4,25 |
| I-25 | 6-$CH_3$ | Cl | $CH(CH_3)CH_2CH_3$ | H | F | F | H | H | F | 187 |
| I-26 | 6-$CH_3$ | Cl | C-$C_5H_9$ | H | F | F | H | H | F | 235 |
| I-27 | 6-$CH_3$ | Cl | (R) $CH(CH_3)CH(CH_3)_2$ | H | F | F | H | H | F | 171 |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-28 | 6-$CH_3$ | Cl | $-(CH_2)_2-CH(CH_3)-(CH_2)_2-$ | | F | F | H | H | F | 0,9 (d,3H); 1,1 (m,2H); 1,6 (m,2H); 2,7 (s,3H); 2,8 (m,2H); 4,1 (m,2H); 6,8 (t,2H); 7,3 (m,1H); 7,7 (t, 1H); 8,1 (d, 1H) |
| I-29 | 6-$CH_3$ | Cl | (S) $CH(CH_3)$ $CH(CH_3)_2$ | H | F | F | H | H | F | 172 |
| I-30 | 6-$CH_3$ | Cl | (S) $CH(CH_3)$ $C(CH_3)_3$ | H | F | F | H | H | F | 187 |
| I-31 | 6-$CH_3$ | Cl | (R) $CH(CH_3)$ $C(CH_3)_3$ | H | F | F | H | H | F | 190 |
| I-32 | 4-$CH_3$ | Cl | (S)$CH(CH_3)$ $CF_3$ | H | F | F | H | H | F | 203-205 |
| I-33 | 4-$CH_3$ | Cl | $CH(CH_3)_2$ | $CH_3$ | F | F | H | H | F | 195-198 |
| I-34 | 4-$CH_3$ | Cl | (S) $CH(CH_3)$ $CH(CH_3)_2$ | H | F | F | H | H | F | 218-220 |
| I-35 | 4-$CH_3$ | Cl | $-(CH_2)_2-CH(CH_3)-(CH_3)_2-$ | | F | F | H | H | F | 168-172 |
| I-36 | 4-$CH_3$ | Cl | (S) $CH(CH_3)$ $C(CH_3)_3$ | H | F | F | H | H | F | 187-189 |
| I-37 | 4-$CH_3$ | Cl | $CH_2CH_3$ | $CH_2CH_3$ | F | F | H | H | F | 145-147 |
| I-38 | 4-$CH_3$ | Cl | $CH_2C(CH_3)_3$ | H | F | F | H | H | F | 192-5 |
| I-39 | 4-$CH_3$ | Cl | $CH_2C(=CH_2)CH_3$ $CH_2CH_3$ | | F | F | H | H | F | 1,1 (t,3H); 1,5 (s,3H); 2,45 (s,3H); 3,4 (q, 2H); 3,9 (m, 2H); 7,8 (m, 2H); 6,8 (t, 2H); 7,2 (m, 1H); 8,2 (s, 1H); 8,7 (m, 1H) |
| I-40 | 4-$CH_3$ | Cl | $CH(CH_3)$ $CH_2CH_3$ | H | F | F | H | H | F | 188-190 |
| I-41 | 4-$CH_3$ | Cl | c-$C_5H_9$ | H | F | F | H | H | F | 195-198 |
| I-42 | 4-$CH_3$ | Cl | $CH(CH_3)_2$ | $CH_3$ | F | F | H | H | F | 185-187 |
| I-43 | 4-$CH_3$ | Cl | $-(CH_2)_2-O-(CH_2)_2-$ | | F | F | H | H | F | 158-162 |
| I-44 | 4-$CH_3$ | Cl | (R) $CH(CH_3)$ $CH(CH_3)_2$ | H | F | F | H | H | F | 215-218 |

(fortgesetzt)

| **Nr.** | **$(R^1)_m$** | **$R^2$** | **$R^3$** | **$R^4$** | **$R^5$** | **$R^6$** | **$R^7$** | **$R^8$** | **$R^9$** | **phys. Daten (Fp.[°C], $^1$H-NMR[ppm]; $logP_{ow}$** |
|---|---|---|---|---|---|---|---|---|---|---|
| I-45 | 4-$CH_3$ | Cl | (R) $CH(CH_3)C(CH_3)_3$ | H | F | F | H | H | F | 184-187 |
| I-46 | H | $CH_3$ | $-(CH_2)_2-CH(CH_3)-(CH_2)_2-$ | | F | F | H | H | F | 112-113 |
| I-47 | H | $OCH_3$ | $CH_2CH_3$ | $CH_2CH_3$ | F | F | H | H | F | 106-108 |
| I-47-1 | H | $CH_3$ | (R)$CH(CH_3)C(CH_3)_3$ | H | F | F | H | H | F | 0,9 (s, 9H); 1,1 (d, 3H); 2,3 (s, 3H); 4,2 (m, 1H); 4,4 (m, 1H); 6,9 (t, 2H); 7,4 (m, 1H); 7,9 (m, 1H); 8,5 (d, 1H); 8,9 (m, 1H) |
| I-48 | H | $CH_3$ | (R)$CH(CH_3)CH(CH_3)_2$ | H | F | F | H | H | F | 0,9 (d, 6H); 1,1 (d, 3H); 1,8 (m, 1H); 2,3 (s, 3H); 4,2 (m, 1H); 4,4 (m, 1H); 6,9 (m, 2H); 7,4 (m, 1H); 7,8 (m, 1H); 8,4 (m, 1H); 8,8 (m, 1H) |
| I-49 | H | $CH_3$ | $CH_2CH_3$ | $CH_2CH_3$ | F | F | H | H | F | 1,0 (t, 6H); 2,3 (s, 3H); 3,4 (q, 4H); 6,8 (m, 2H); 7,4 (m, 1H); 7,8 (m, 1H); 8,4 (m, 1H); 8,8 (m, 1H) 0,9 (d, 6H); 1,1 (d, 3H); 1,8 (m, 1H); 2,3 (s, 3H); 4,2 (m, 1H); 4,4 (m, 1H); 6,8 (m, 2H); 7,4 (m, 1H); 7,8 (m, 1H); 8,4 (m, 1H); 8,8 (m, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1H$-NMR[ppm]; $logP_{ow}$ |
|---|---|---|---|---|---|---|---|---|---|---|
| I-50 | H | $CH_3$ | (S)$CH(CH_3)CH(CH_3)_2$ | H | F | F | H | H | F | 0,9 (d, 6H); 1,1 (d, 3H); 1,8 (m, 1H); 2,3 (s, 3H); 4,2 (m, 1H); 4,4 (m, 1H); 6,8 (m, 2H); 7,4 (m, 1H); 7,8 (m, 1H); 8,4 (m, 1H); 8,8 (m, 1H) |
| I-51 | H | Cl | $CH(CH_3)CF_3$ | H | Cl | F | H | H | H | 150 |
| I-52 | H | Cl | $CH_2C(=CH_2)CH_3$ | $CH_2CH_3$ | F | F | H | H | F | 1,1 (t, 3H); 1,5 (s, 3H); 3,4 (q, 2H); 3,9 (s, 2H); 4,8 (s, 2H); 6,8 (t, 2H); 7,4 (m, 1H); 7,8 (t, 1H); 8,4 (d, 1H); 8,8 (d, 1H) |
| I-53 | H | Cl | $CH(CH_3)CH_2CH_3$ | H | F | F | H | H | F | 0,9 (d, 6H); 1,1 (d, 3H); 1,8 (m, 1H); 2,3 (s, 3H); 4,2 (m, 1H); 4,4 (m, 1H); 6,9 (m, 2H); 7,4 (m, 1H); 7,8 (m, 1H); 8,4 (m, 1H); 8,8 (m, 1H) |
| I-54 | H | Cl | $CH(CH_3)CH_2CH_3$ | H | F | F | H | H | F | 172-175 |
| I-55 | H | Cl | $CH(CH_3)_2$ | $CH_3$ | F | F | H | H | F | 158 |
| I-56 | H | Cl | $CH_2CF_3$ | H | F | F | H | H | F | 200 |
| I-57 | H | Cl | $(CH_2)_2CH(CH_3)(CH_2)_2$ | | F | F | H | H | F | 0,9 (d, 3H); 1,1 (m, 2H); 1,7 (m, 3H); 3,0 (t, 2H); 4,5 (d, 2H); 6,8 (t, 2H); 7,4 (m, 1H); 7,8 (m, 1H); 8,4 (m, 1H); 8,8 (m, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-58 | H | Cl | $(CH_2)_2CH(CH_3)(CH_2)_2$ | | F | F | H | H | $OCH_3$ | 133-135 |
| I-59 | H | Cl | $CH(CH_3)_2$ | H | F | F | H | H | $OCH_3$ | 173-175 |
| I-60 | H | Cl | (R)$CH(CH_3)CH(CH_3)_2$ | H | F | F | H | H | $OCH_3$ | 147-149 |
| I-61 | H | Cl | (R)$CH(CH_3)C(CH_3)_3$ | H | F | F | H | H | $OCH_3$ | 179-181 |
| I-62 | H | Cl | $CH(CH_3)CH_2CH_3$ | H | F | F | H | H | $OCH_3$ | 142-144 |
| I-63 | H | Cl | $CH_2C(=CH_2)CH_3$ | $CH_2CH_3$ | F | F | H | H | $OCH_3$ | 135-137 |
| I-64 | H | Cl | $CH_2CH_3$ | $CH_2CH_3$ | F | F | H | H | $OCH_3$ | logP 5.0 |
| I-65 | H | Cl | $CH_2CH_3$ | H | F | F | H | H | $OCH_3$ | logP 4.0 |
| I-66 | H | Cl | c-$C_5H_9$ | H | F | F | H | H | $OCH_3$ | 173-175 |
| I-67 | H | Cl | (S)$CH(CH_3)CH(CH_3)_2$ | H | F | F | H | H | $OCH_3$ | 145-146 |
| I-68 | H | Cl | (S)$CH(CH_3)C(CH_3)_3$ | H | F | F | H | H | $OCH_3$ | 185-187 |
| I-69 | H | Cl | $(CH_2)_2CH(CH_3)(CH_2)_2$ | | F | F | H | H | OH | 185-187 |
| I-70 | H | Cl | $CH(CH_3)_2$ | H | F | F | H | H | OH | log.P 3.0 |
| I-71 | H | Cl | (R)$CH(CH_3)CH(CH_3)_2$ | H | F | F | H | H | OH | 173-175 |
| I-72 | H | Cl | (R)$CH(CH_3)C(CH_3)_3$ | H | F | F | H | H | OH | 175-177 |
| I-73 | H | Cl | $CH_2C(=CH_2)CH_3$ | $CH_2CH_3$ | F | F | H | H | OH | 135-137 |
| I-74 | H | Cl | (S)$CH(CH_3)CH(CH_3)_2$ | H | F | F | H | H | OH | 110-112 |
| I-75 | H | Cl | (S)$CH(CH_3)C(CH_3)_3$ | H | F | F | H | H | OH | 109-110 |
| I-76 | H | Cl | c-$C_5H_9$ | H | F | F | H | H | OH | 128-130 |
| I-77 | H | Cl | $CH_2CH(CH_3)_2$ | $CH_3$ | F | F | H | H | OH | 153-155 |
| I-78 | H | Cl | $CH_2CH(CH_3)_2$ | $CH_2CH(CH_3)_2$ | F | F | H | H | OH | 0,8 (d, 12H); 1,8 (m, 2H); 3,3 (m, 4H); 6,8 (d, 2H); 7,6 (m, 1H); 8,0 (m, 1H); 8,6 (d, 1H); 8,9 (m, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-79 | H | Cl | $CH_2CH(CH_3)_2$ | $CH_2CH_3$ | F | F | H | H | OH | 0,8 (d, 6H); 1,0 (t, 3H); 1,9 (m, 1H); 3,3 (d, 2H); 3,5 (q, 2H); 6,8 (d, 2H); 7,6 (m, 1H); 8,0 (m, 1H); 8,6 (d, 1H); 8,9 (m, 1H) |
| I-80 | H | Cl | $(CH_2)_2CH(CH_3)(CH_2)_2$ | | F | F | H | H | H | 0,9 (d, 3H); 1,1 (m, 2H); 1,7 (m, 3H); 3,0 (t, 2H); 4,5 (d, 2H); 6,8 (t, 2H); 7,5 (m, 1H); 8,0 (m, 1H); 8,5 (t, 1H); 8,8 (d, 1H); 9,4 (d, 1H) |
| I-81 | H | Cl | $CH_2CH_3$ | $CH_2CH_3$ | F | F | H | H | H | logP 4.7 |
| I-82 | H | Cl | (S)$CH(CH_3)CH(CH_3)_2$ | H | F | F | H | H | H | logP 5.0 |
| I-83 | H | Cl | $CH(CH_3)_2$ | H | F | F | H | H | H | logP 4.1 |
| I-84 | H | Cl | (R)$CH(CH_3)CH(CH_3)_2$ | H | F | F | H | H | H | logP 5.0 |
| I-85 | H | Cl | $(CH_2)_2CH(CH_3)(CH_2)_2$ | | $CH_3$ | H | H | H | $CH_3$ | 90-94 |
| I-86 | H | Cl | $CH(CH_3)_2$ | H | $CH_3$ | H | H | H | $CH_3$ | 1,1 (d, 6H); 2,1 (s, 3H); 2,4 (s, 3H); 4,5 (m, 1H); 4,9 (m, 1H); 7,0 (d, 1H); 7,1 (d, 1H); 7,8 (m, 1H); 8,3 (t, 1H); 8,7 (d, 1H); 9,2 (m, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-87 | H | Cl | c-$C_5H_9$ | H | $CH_3$ | H | H | H | $CH_3$ | 1,2 (m, 2H); 1,6 (m, 2H); 1,7 (m, 2H); 2,1 (s, 3H); 2,2 (m, 2H); 2,5 (s, 3H); 4,7 (m, 1H); 5,0 (m, 1H); 7,0 (d, 1H); 7,1 (d, 1H); 7,8 (t, 1H); 8,2 (t, 1H); 8,7 (d, 1H); 9,2 (m, 1H) |
| I-88 | H | Cl | (S)CH($CH_3$) CH $(CH_3)_2$ | H | $CH_3$ | H | H | H | $CH_3$ | 0,9 (dd, 6H); 1,1 (d, 3H); 1,8 (m, 1H); 2,1 (d, 3H); 2,4 (s, 3H); 4,5 (m, 2H); 7,0 (d, 1H); 7,1 (d, 1H); 7,8 (m, 1H); 8,3 (m, 1H); 8,7 (m, 1H); 9,2 (m, 1H) |
| I-89 | H | Cl | (R)CH($CH_3$) CH $(CH_3)_2$ | H | $CH_3$ | H | H | H | $CH_3$ | 0,9 (dd, 6H); 1,1 (d, 3H); 1,8 (m, 1H); 2,1 (d, 3H); 2,4 (s, 3H); 4,5 (m, 1H); 4,8 (m, 1H); 7,0 (d, 1H); 7,1 (m, 1H); 7,8 (m, 1H); 8,3 (m, 1H); 8,7 (m, 1H); 9,2 (m, 1H) |
| I-90 | H | Cl | $CH_2CH_3$ | H | $CH_3$ | H | H | H | $CH_3$ | logP 4.6 |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-91 | H | Cl | $CH_2CH_3$ | $CH_2CH_3$ | $CH_3$ | H | H | H | $CH_3$ | 1,0 (t, 6H); 2,0 (s, 3H); 2,4 (s, 3H); 3,4 (m, 4H); 4,5 (m, 2H); 7,1 (m, 3H); 7,4 (m, 1H); 7,8 (t, 1H); 8,4 (d, 1H); 8,8 (d, 1H) |
| I-92 | H | Cl | (R)$CH(CH_3)$ C $(CH_3)_3$ | H | $CH_3$ | H | H | H | $CH_3$ | 0,8 (s, 9H); 1,1 (dd, 3H); 2,4 (s, 3H); 4,4 (m, 1H); 4,6 (t, 1H); 7,1 (m, 3H); 7,4 (m, 1H); 7,8 (t, 1H); 8,4 (d, 1H); 8,8 (m, 1H) |
| I-93 | H | Cl | $(CH_2)_2CH$ $(CH_3)$ $(CH_2)_2$ | | F | F | H | H | CN | 0,9 (d, 3H); 1,1 (m, 2H); 1,6 (m, 3H); 2,8 (m, 2H); 3,9 (m, 2H); 7,3 (m, 2H); 7,4 (m, 1H); 7,8 (m, 1H); 8,4 (d, 1H); 8,8 (m, 1H) |
| I-94 | H | Cl | $CH(CH_3)_2$ | H | F | H | H | H | $CH_3$ | logP 4.8 |
| I-95 | H | Cl | $CH(CH_3)$ $(CH_2)_2$ | H | F | H | H | H | $CH_3$ | 1,2 (d, 3H); 1,6 (m, 2H); 1,8 (m, 2H); 2,4 (s, 3H); 3,0 (m, 2H); 4,8 (m, 1H); 7,0 (m, 3H); 7,9 (m, 1H); 8,4 (q, 1H); 8,7 (d, 1H); 9,2 (m, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-96 | H | Cl | (S)CH($CH_3$) CH $(CH_3)_2$ | H | F | H | H | H | $CH_3$ | 0,9 (dd, 6H); 1,1 (d, 3H); 1,8 (m, 1H); 2,4 (s, 3H); 4,8 (m, 2H); 7,1 (m, 3H); 7,8 (t, 1H); 8,4 (t, 1H); 8,7 (d, 1H); 9,2 (d, 1H) |
| I-97 | H | Cl | (R)CH($CH_3$) CH $(CH_3)_2$ | H | F | H | H | H | $CH_3$ | 0,9 (dd, 6H); 1,1 (d, 3H); 1,8 (m, 1H); 2,4 (s, 3H); 4,5 (m, 1H); 4,6 (m, 1H); 7,1 (m, 3H); 7,6 (t, 1H); 8,1 (t, 1H); 8,6 (d, 1H); 9,0 (d, 1H) |
| I-98 | H | Cl | (S)CH($CH_3$) C $(CH_3)_3$ | H | F | H | H | H | $CH_3$ | 0,8 (s, 9H); 1,1 (dd, 3H); 2,4 (s, 3H); 4,4 (m, 1H); 4,6 (t, 1H); 7,1 (m, 3H); 7,4 (m, 1H); 7,8 (t, 1H); 8,4 (d, 1H); 8,8 (m, 1H) |
| I-99 | H | Cl | $(CH_2)_2$CH ($CH_3$) $(CH_2)_2$ | | F | H | H | H | $CH_3$ | 172-174 |
| I-100 | H | Cl | CH($CH_3$) $CH_2CH_3$ | H | F | H | H | H | $CH_3$ | 0,9 (m, 3H); 1,2 (d, 3H); 2,4 (s, 3H); 4,6 (m; 1H); 4,7 (m, 1H); 7,1 (m, 3H); 7,8 (t, 1H); 8,2 (t, 1H); 8,7 (d, 1H); 9,1 (m, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-101 | H | Cl | $CH_2CH_3$ | $CH_2CH_3$ | F | H | H | H | $CH_3$ | 1,0 (t, 6H); 2,4 (s, 3H); 3,5 (m, 4H); 7,1 (m, 3H); 7,8 (t, 1H); 8,4 (t, 1H); 8,7 (d, 1H); 9,2 (m, 1H) |
| I-102 | H | Cl | c-$C_5H_9$ | H | F | H | H | H | $CH_3$ | 1,4 (m, 2H); 1,6 (m, 4H); 2,2 (m, 2H); 4,8 (m, 2H); 7,1 (m, 3H); 7,8 (t, 1H); 8,2 (t, 1H); 8,7 (d, 1H); 9,1 (m, 1H) |
| I-103 | H | Cl | $CH_2C(=CH_2)CH_3$ | $CH_2CH_3$ | F | H | H | H | $CH_3$ | 1,0 (t, 3H); 1,4 (s, 3H); 2,4 (s, 3H); 3,4 (m, 2H); 3,9 (m, 2H); 4,8 (m, 2H); 7,0 (m, 2H); 7,1 (t, 1H); 7,4 (m, 1H); 7,8 (t, 1H); 8,4 (d, 1H); 8,8 (d, 1H) |
| I-104 | H | Cl | $CH(CH_3)C(CH_3)_3$ | H | F | H | H | H | $CH_3$ | 0,8 (s, 9H); 1,3 (s, 3H); 2,4 (s, 3H); 4,4 (m, 1H); 4,6 (m, 1H); 7,1 (m, 3H); 7,4 (m, 1H); 7,8 (t, 1H); 8,4 (d, 1H); 8,8 (d, 1H) |
| I-105 | H | Cl | $CH(CH_3)_2$ | H | $CH_3$ | H | H | H | F | 1,2 (d, 6H); 2,1 (s, 3H); 4,4 (m, 1H); 4,8 (m, 1H); 7,1 (m, 3H); 7,6 (m, 1H); 8,1 (m, 1H); 8,6 (m, 1H); 9,1 (m, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1H$-NMR[ppm]; $logP_{ow}$ |
|---|---|---|---|---|---|---|---|---|---|---|
| I-106 | H | Cl | $CH(CH_3)(CH_2)_3$ | H | $CH_3$ | H | H | H | F | 1,2 (m, 3H); 1,6 (m, 2H); 1,8 (m, 2H); 2,1 (s, 3H); 2,9 (m, 2H); 5,1 (m, 1H); 7,0 (m, 2H); 7,3 (m, 1H); 7,8 (t, 1H); 8,3 (t, 1H); 8,7 (d, 1H); 9,3 (m, 1H) |
| I-107 | H | Cl | (S)$CH(CH_3)CH(CH_3)_2$ | H | $CH_3$ | H | H | H | F | 51-52 |
| I-108 | H | Cl | (R)$CH(CH_3)CH(CH_3)_2$ | H | $CH_3$ | H | H | H | F | 0,8 (d, 6H); 1,1 (d, 3H); 1,8 (m, 1H); 2,1 (s, 3H); 4,3 (m, 2H); 7,1 (m, 3H); 7,4 (m, 1H); 7,8 (t, 1H); 8,4 (m, 1H); 8,8 (d, 1H) |
| I-109 | H | Cl | $(CH_2)_2CH(CH_3)(CH_2)_2$ | | $CH_3$ | H | H | H | F | 0,9 (d, 3H); 1,0 (m, 2H); 1,6 (m, 4H); 2,2 (s, 3H); 2,8 (t, 2H); 4,2 (m, 1H); 4,3 (m, 1H); 7,0 (m, 2H); 7,1 (m, 1H); 7,8 (t, 1H); 8,3 (t, 1H); 8,7 (d, 1H); 9,2 (m, 1H) |
| I-110 | H | Cl | $CH(CH_3)CH_2CH_3$ | H | $CH_3$ | H | H | H | F | 0,9 (t, 3H); 1,2 (d, 3H); 1,4 (m, 2H); 2,2 (s, 3H); 4,3 (m, 2H); 7,1 (m, 3H); 7,4 (m, 1H); 7,8 (t, 1H); 8,4 (d, 1H); 8,8 (m, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1H$-NMR[ppm]; $logP_{ow}$ |
|---|---|---|---|---|---|---|---|---|---|---|
| I-111 | H | Cl | $CH_2CH_3$ | $CH_2CH_3$ | $CH_3$ | H | H | H | F | 1,0 (t, 6H); 2,2 (s, 3H); 3,3 (m, 4H); 7,0 (m, 2H); 7,2 (m, 1H); 7,4 (m, 1H); 7,8 (t, 1H); 8,4 (d, 1H); 8,8 (m, 1H) |
| I-112 | H | Cl | c-$C_5H_9$ | H | $CH_3$ | H | H | H | F | 1,3 (m, 2H); 1,6 (m, 4H); 2,2 (m, 5H); 4,6 (m, 1H); 5,0 (m, 1H); 7,1 (m, 3H); 7,8 (t, 1H); 8,3 (t, 1H); 8,7 (d, 1H); 9,2 (m, 1H) |
| I-113 | H | Cl | (R)CH($CH_3$) C ($CH_3$)$_3$ | H | $CH_3$ | H | H | H | F | 162-164 |
| I-114 | H | Cl | $CH_2CH_3$ | H | $CH_3$ | H | H | H | F | logP 4.1 |
| I-115 | 4-$CH_3$ | Cl | $CH_2CH$ $(CH_3)_2$ | $CH_2CH_3$ | F | F | H | H | F | 0,8 (t, 6H); 1,0 (t, 3H); 2,5 (s, 3H); 3,2 (m, 2H); 3,4 (q, 2H); 6,8 (t, 2H); 7,2 (m, 1H); 8,2 (s, 1H); 8,7 (d, 1H) |
| I-116 | 3-$CH_3$ | Cl | (S)CH($CH_3$) C ($CH_3$)$_3$ | H | F | F | H | H | F | 128-132 |
| I-117 | 3-$CH_3$ | Cl | $CH_2CH_3$ | $CH_2CH_3$ | F | F | H | H | F | logP 4.6 |
| I-118 | 3-$CH_3$ | Cl | $CH_2C(CH_3)_3$ | H | F | F | H | H | F | 198 |
| I-119 | 3-$CH_3$ | Cl | $CH(CH_3)_2$ | $CH_3$ | F | F | H | H | F | 94-96 |
| I-120 | 3-$CH_3$ | Cl | $CH_2CH$ $(CH_3)_2$ | $CH_2CH_3$ | F | F | H | H | F | 0,8 (d, 6H); 1,0 (t, 3H); 1,9 (m, 1H); 2,5 (s, 3H); 3,1 (d, 2H); 3,3 (q, 2H); 6,8 (t, 2H); 7,2 (m, 1H); 7,6 (d, 1H); 8,6 (d, 1H) |

(fortgesetzt)

| Nr. | $(R^1)_m$ | $R^2$ | $R^3$ | $R^4$ | $R^5$ | $R^6$ | $R^7$ | $R^8$ | $R^9$ | phys. Daten (Fp.[°C], $^1$H-NMR[ppm]; $logP_{ow}$ |
|---|---|---|---|---|---|---|---|---|---|---|
| I-121 | 3-$CH_3$ | Cl | $CH_2C(=CH_2)CH_3$ | $CH_2CH_3$ | F | F | H | H | F | 1,0 (t, 3H); 1,5 (s, 3H); 2,5 (s, 3H); 3,3 (q, 2H); 3,8 (s, 2H); 4,8 (d, 2H); 6,8 (t, 2H); 7,2 (m, 1H); 7,6 (d, 1H); 8,6 (d, 1H) |
| I-122 | 3-$CH_3$ | Cl | $(CH_2)_2O(CH_2)_2$ | | F | F | H | H | F | 122-127 |
| I-123 | 3-$CH_3$ | Cl | $CH(CH_3)CH_2CH_3$ | H | F | F | H | H | F | 154 |
| I-124 | 3-$CH_3$ | Cl | (S)$CH(CH_3)CH(CH_3)_2$ | H | F | F | H | H | F | 161 |
| I-125 | 3-$CH_3$ | Cl | $(CH_2)_2CH(CH_3)(CH_2)_2$ | | F | F | H | H | F | 130-132 |
| I-125 | 3-$CH_3$ | Cl | c-$C_5H_9$ | H | F | F | H | H | F | 161-163 |
| I-127 | 3-$CH_3$ | Cl | $CH(CH_3)_2$ | | F | F | H | H | F | 144-148 |
| I-128 | 3-$CH_3$ | Cl | (R)$CH(CH_3)C(CH_3)_3$ | H | F | F | H | H | F | 134-138 |
| I-129 | 3-$CH_3$ | Cl | (R)$CH(CH_3)CH(CH_3)_2$ | H | F | F | H | H | F | 159 |
| I-130 | 3-$CH_3$ | Cl | (S)$CH(CH_3)CF_3$ | H | F | F | H | H | F | 172 |

Die Bestimmung der Lipophilieparameter $logP_{ow}$ (Tabelle I) erfolgte gemäß der OECD-Prüfrichtlinie nach der RP-HPLC-Laufzeitmethode.

Dazu wurde eine Korrelationskurve logk'/$logP_{ow}$ basierend auf zehn Referenzsubstanzen erstellt und mit Hilfe der durch die Extraktionsmethode ermittelten Lipophilieparameter von acht Vergleichssubstanzen validiert.

Als stationäre Phase wurde eine handelsübliche $C_{18}$-Umkehrphase verwendet. Die chromatographische Trennung erfolgte mit Methanol und einer Pufferlösung als mobile Phase bei pH 7,4 unter isokratischen Bedingungen.

Die Retentionszeiten der Refernzen $t_R$ wurden gemäß Gleichung Φ in die Kapazitätsfaktoren k' überführt, wobei to als Retentionszeit des an der $C_{18}$-Umkehrphase unretardierten Lösungsmittels die Totzeit des chromatographischen Systems darstellt:

$$k' = \frac{t_R - t_0}{t_0} \qquad \Phi$$

Die lineare Korrelation der logk'- mit den im Anhang zur Richtlinie 92/69/EWG publizierten $logP_{ow}$-Werten der Referenzen liefert die Korrelationskurve durch lineare Regression.

Die Lipophilieparameter $logP_{ow}$ der Analyten wurden nach Berechnung des logarithmierten Kapazitätsfaktors logk' aus der Korrelationskurve der Referenzen interpoliert.

Die Valierung der beschriebenen RP-HPLC-Analysenmethode und der verwendeten Referenzen erfolgt mit

Hilfe von acht Vergleichswirkstoffen, deren Verteilungsverhalten mit Hilfe der Extraktionsmethode bestimmt worden ist.

Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

Anwendungsbeispiel 1 - Wirksamkeit gegen *Alternaria solani* an Tomaten

Blätter von Topfpflanzen der Sorte "Große Fleischtomate St. Pierre" wurden mit einer wäßrigen Suspension, die aus einer Stammlösung aus 10 % Wirkstoff, 63 % Cyclohexanon und 27 % Emulgiermittel angesetzt wurde, bis zur Tropfnässe besprüht. Am folgenden Tag wurden die Blätter mit einer wäßrigen Zoosporenaufschwemmung von *Alternaria solani* in 2 % Biomalzlösung mit einer Dichte von 0,17 x $10^6$ Sporen/ml infiziert. Anschließend wurden die Pflanzen in einer wasserdampfgesättigten Kammer bei Temperaturen zwischen 20 und 22˚C aufgestellt. Nach 5 Tagen hatte sich die Krautfäule auf den unbehandelten, jedoch infizierten Kontrollpflanzen so stark entwickelt, daß der Befall visuell in % ermittelt werden konnte.

In diesem Test zeigten die mit 63 ppm der Wirkstoffe I-1 bis I-7, I-9 bis I-16, I-20, I-24, I-25, I-32, I-33, I-51, I-54, I-58, I-59, I-62, I-63, I-64, I-66, I-67, I-68 und I-94 behandelten Pflanzen maximal 3 % Befall, während die unbehandelten Pflanzen zu 100 % befallen waren.

Anwendungsbeispiel 2: Wirksamkeit gegen *Botrytis cinerea* an Paprikablättern

Paprikasämlinge der Sorte "Neusiedler Ideal Elite" wurden, nachdem sich 4 bis 5 Blätter gut entwickelt hatten, mit einer wässrigen Wirkstoffaufbereitung, die aus einer Stammlösung bestehend aus 10 % Wirkstoff, 85 % Cyclohexanon und 5 % Emulgiermittel angesetzt wurde, bis zur Tropfnässe besprüht. Am nächsten Tag wurden die behandelten Pflanzen mit einer Sporensuspension von *Botrytis cinerea*, die 1,7 x $10^6$ Sporen/ml in einer 2 %igen wässrigen Biomalzlösung enthielt, inokuliert. Anschließend wurden die Versuchspflanzen in eine Klimakammer mit 22 bis 24˚C und hoher Luftfeuchtigkeit gestellt. Nach 5 Tagen konnte das Ausmaß des Pilzbefall auf den Blättern visuell in % ermittelt werden.

In diesem Test zeigten die mit 250 ppm der Wirkstoffe I-1 bis I-7, I-9 bis I-14, I-20, I-24, I-32 bis I-41, I-43, I-51, I-53, I-55, I-56, I-57, I-58, I-59, I-62, I-63, I-64, I-66, I-67, I-68, I-82, I-83, I-86, I-87, I-88, I-90 und I-115 bis zu 5 % Befall, während die unbehandelten Pflanzen zu 90 % befallen waren.

Anwendungsbeispiel 3: Protektive Wirksamkeit gegen den durch *Sphaerotheca fuliginea* verursachten Gurkenmehltau

Blätter von in Töpfen gewachsenen Gurkenkeimlingen der Sorte "*Chinesische Schlange*" wurden im Keimblattstadium mit wässriger Wirkstoffaufbereitung, die aus einer Stammlösung bestehend aus 10 % Wirkstoff, 85 % Cyclohexanon und 5 % Emulgiermittel angesetzt wurde, bis zur Tropfnässe besprüht. 20 Stunden nach dem Antrocknen des Spritzbelages wurden die Pflanzen mit einer wässrigen Sporensuspension des Gurkenmehltaus (*Sphaerotheca fuliginea*) inokuliert. Anschließend wurden die Pflanzen im Gewächshaus bei Temperaturen zwischen 20 und 24˚C und 60 bis 80 % relativer Luftfeuchtigkeit für 7 Tage kultiviert. Dann wurde das Ausmaß der Mehltauentwicklung visuell in %-Befall der Keimblattfläche ermittelt.

In diesem Test zeigten die mit 63 ppm der Wirkstoffe I-1, I-3, I-5 bis I-10, I-12, I-13, I-16, I-18, I-21, I-32, I-33, I-51, I-52, I-54, I-55, I-56, I-58, I-59, I-62, I-63, I-64, I-66, I-67, I-68 und I-83 maximal 10 % Befall, während die unbehandelten Pflanzen zu 100 % befallen waren.

Anwendungsbeispiel 4 - Wirksamkeit gegen die Netzfleckenkrankheit der Gerste

Blätter von in Töpfen gewachsenen Gerstenkeimlingen der Sorte "Igri" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit einer wäßrigen Sporensuspension von *Pyrenophora [syn. Drechslera] teres*, dem Erreger der Netzfleckenkrankheit inokuliert. Anschließend wurden die Versuchspflanzen im Gewächshaus bei Temperaturen zwischen 20 und 24˚C und 95 bis 100 % relativer Luftfeuchtigkeit aufgestellt. Nach 6 Tagen wurde das Ausmaß der Krankheitsentwicklung visuell in % Befall

der gesamten Blattfläche ermittelt.

In diesem Test zeigten die mit 63 ppm der Wirkstoffe I-1 bis I-6, I-10, I-12 bis I-14, I-32, I-33, I-38, I-43, I-51, I-56, I-64 und I-86 behandelten Pflanzen nicht über 15 % Befall, während die unbehandelten Pflanzen zu 90 % befallen waren.

## Patentansprüche

**1.** 2-(2-Pyridyl)-5-phenyl-6-aminopyrimidine der Formel I,

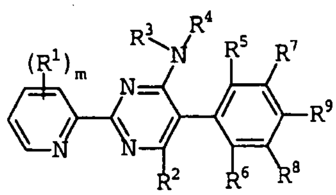

I

in der die Substituenten und der Index folgende Bedeutung haben:

$R^1$ Halogen, Hydroxy, Cyano, Oxo, Nitro, Amino, Mercapto, $C_1$-$C_6$-Alkyl, $C_1$-$C_6$-Halogenalkyl, $C_2$-$C_6$-Alkenyl, $C_2$-$C_6$-Alkinyl, $C_3$-$C_6$-Cycloalkyl, $C_1$-$C_6$-Alkoxy, $C_1$-$C_6$-Halogenalkoxy, Carboxyl, $C_1$-$C_7$-Alkoxycarbonyl, Carbamoyl, $C_1$-$C_7$-Alkylaminocarbonyl, $C_1$-$C_6$-Alkyl-$C_1$-$C_6$-alkylamincarbonyl, Morpholinocarbonyl, Pyrrolidinocarbonyl, $C_1$-$C_7$-Alkylcarbonylamino, $C_1$-$C_6$-Alkylamino, Di-($C_1$-$C_6$-alkyl)amino, $C_1$-$C_6$-Alkylthio, $C_1$-$C_6$-Alkylsulfinyl, $C_1$-$C_6$-Alkylsulfonyl, Hydroxysulfonyl, Aminosulfonyl, $C_1$-$C_6$-Alkylaminosulfonyl, Di-($C_1$-$C_6$-alkyl)aminosulfonyl;

m 0, 1, 2, 3 oder 4;

$R^2$ Halogen, Cyano, $C_1$-$C_6$-Alkyl, $C_1$-$C_6$-Halogenalkyl, $C_1$-$C_6$-Alkoxy, $C_1$-$C_6$-Halogenalkoxy oder $C_3$-$C_6$-Alkenyloxy;

$R^3$, $R^4$ unabhängig voneinander Wasserstoff, $C_1$-$C_6$-Alkyl, $C_1$-$C_6$-Halogenalkyl, $C_3$-$C_6$-Cycloalkyl, $C_3$-$C_6$-Halogencycloalkyl, $C_2$-$C_6$-Alkenyl, $C_2$-$C_6$-Halogenalkenyl, $C_3$-$C_6$-Cycloalkenyl, $C_2$-$C_6$-Alkinyl, $C_2$-$C_6$-Halogenalkinyl oder $C_3$-$C_6$-Cycloalkinyl,

$R^3$ und $R^4$ können auch zusammen mit dem Stickstoffatom, an das sie gebunden sind, einen fünf- oder sechsgliedrigen Ring bilden, der durch ein Atom aus der Gruppe O, N oder S unterbrochen sein und/oder einen oder mehrere Substituenten aus der Gruppe Halogen, $C_1$-$C_6$-Alkyl, $C_1$-$C_6$-Halogenalkyl oder Oxy-$C_1$-$C_3$-alkylenoxy tragen kann oder in dem zwei benachbarte C-Atome oder ein N- und ein benachbartes C-Atom durch eine $C_1$-$C_4$-Alkylenkette verbunden sein können;

$R^5$ Halogen, $C_1$-$C_6$-Alkyl oder $C_1$-$C_6$-Halogenalkyl;

$R^6$ Wasserstoff oder eine der bei $R^5$ genannten Gruppen;

$R^7$, $R^8$ unabhängig voneinander Wasserstoff, Halogen, $C_1$-$C_6$-Alkyl oder $C_1$-$C_6$-Halogenalkyl;

$R^9$ Wasserstoff, Halogen, Hydroxy, Cyano, $C_1$-$C_6$-Alkyl, $C_1$-$C_6$-Alkoxy, $C_3$-$C_6$-Cycloalkoxy, $C_1$-$C_6$-Halogenalkoxy, $C_1$-$C_6$-Alkoxycarbonyl oder $C_1$-$C_6$-Alkylaminocarbonyl.

**2.** Verbindungen der Formel I gemäß Anspruch 1, wobei m für null oder 1, 2 oder 3 steht und $R^1$ folgende Bedeutung hat:

Halogen, Hydroxy, Cyano, Nitro, Amino, Mercapto, $C_1$-$C_6$-Alkyl, $C_1$-$C_6$-Halogenalkyl, $C_2$-$C_6$-Alkenyl, $C_2$-$C_6$-Alkinyl, $C_3$-$C_6$-Cycloalkyl, $C_1$-$C_6$-Alkoxy, $C_1$-$C_6$-Halogenalkoxy, Carboxyl, $C_1$-$C_7$-Alkoxycarbonyl, Carbamoyl, $C_1$-$C_7$-Alkylaminocarbonyl, $C_1$-$C_6$-Alkyl-$C_1$-$C_6$-alkylamincarbonyl, Morpholinocarbonyl, Pyrrolidinocarbonyl, $C_1$-$C_7$-Alkylcarbonylamino, $C_1$-$C_6$-Alkylamino, Di-($C_1$-$C_6$-alkyl)amino, $C_1$-$C_6$-Alkylthio, $C_1$-$C_6$-Alkylsulfinyl, $C_1$-$C_6$-Alkylsulfonyl, Hydroxysulfonyl, Aminosulfonyl, $C_1$-$C_6$-Alkylaminosulfonyl oder Di-($C_1$-$C_6$-alkyl)aminosulfonyl;

**3.** Verbindungen der Formel I gemäß Anspruch 2, wobei die Variablen folgende Bedeutung haben

$R^2$ Halogen, $C_1$-$C_6$-Alkyl oder $C_1$-$C_6$-Alkoxy;

$R^3$, $R^4$ unabhängig voneinander Wasserstoff, $C_1$-$C_6$-Alkyl, $C_1$-$C_6$-Halogenalkyl, $C_3$-$C_6$-Cycloalkyl oder $C_2$-$C_6$-Alkenyl;

$R^3$ und $R^4$ können auch zusammen mit dem Stickstoffatom, an das sie gebunden sind, einen fünf- oder sechsgliedrigen Ring bilden, der durch ein Sauerstoffatom unterbrochen sein und einen $C_1$-$C_6$-Alkylsubstituenten tragen kann;
$R^5$, $R^6$ unabhängig voneinander Halogen;
$R^7$, $R^8$ unabhängig voneinander Wasserstoff oder Halogen;
$R^9$ Wasserstoff, Halogen, Hydroxy, $C_1$-$C_6$-Alkoxy oder $C_1$-$C_6$-Alkoxycarbonyl.

**4.** Verbindungen der Formel I gemäß Ansprüchen 1 bis 3, wobei $R^2$ Chlor bedeutet.

**5.** Verbindungen der Formel I gemäß Ansprüchen 1 bis 4, wobei die Kombination der Substituenten $R^5$ bis $R^9$ für folgende Bedeutungen steht: 2-Methyl,4-Fluor; 2-Fluor,4-Methyl; 2,4-Dimethyl; 2-Chlor,6-Fluor; 2,6-Difluor; 2,6-Dichlor; 2-Methyl,6-Fluor; 2,4,6-Trifluor; 2,6-Difluor; 4-Methoxy und Pentafluor.

**6.** Verfahren zur Herstellung von 5-Phenylpyridinen der Formel I gemäß Ansprüchen 1 bis 5, in denen $R^2$ für Chlor steht, **dadurch gekennzeichnet, daß** man 2-Pyridylamidine der Formel II,

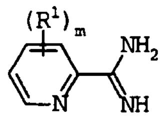

II

mit Phenylmalonaten der Formel III,

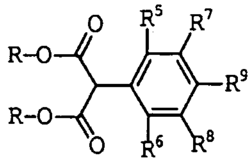

III

in der R für $C_1$-$C_6$-Alkyl steht, zu Verbindungen der Formel IV,

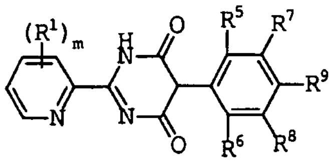

IV

umsetzt, welche mit Chlorierungsmitteln in die Dichlorpyrimidine der Formel V

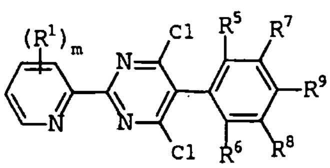

V

überführt werden, die mit Aminen der Formel VI

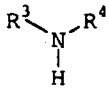

VI

zu den Pyrimidinderivaten der Formel I, in der $R^2$ für Chlor steht, umgesetzt werden.

**7.** Verfahren zur Herstellung von 5-Phenylpyridinen der Formel I gemäß Ansprüchen 1 bis 5, in denen $R^2$ für $C_1$-$C_6$-Alkyl oder $C_1$-$C_6$-Halogenalkyl steht, **dadurch gekennzeichnet, daß** man ein 2-Pyridylamidin der Formel II gemäß Anspruch 6, mit Phenyl-βketoestern der Formel VII,

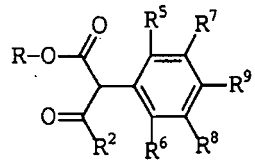

VII

in der R für $C_1$-$C_6$-Alkyl steht, zu Verbindungen der Formel IVa

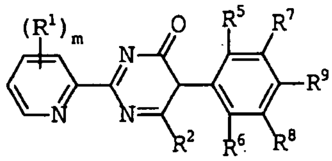

IVa

umsetzt, welche mit Chlorierungsmitteln in die Chlorpyrimidine der Formel Va

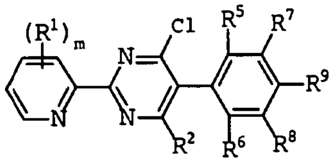

Va

überführt werden, die mit Aminen VI gemäß Anspruch 6 zu den Pyrimidinderivaten der Formel I, in der $R^2$ für $C_1$-$C_6$-Alkyl oder $C_1$-$C_6$-Halogenalkyl steht, umgesetzt werden.

**8.** Zwischenprodukte der Formeln IV und V gemäß Anspruch 6, wobei die Kombination der Substituenten $R^5$ bis $R^9$ die Bedeutungen gemäß Anspruch 5 aufweisen.

**9.** Zwischenprodukte der Formeln IVa und Va gemäß Anspruch 7, wobei die Kombination der Substituenten $R^5$ bis $R^9$ die Bedeutungen gemäß Anspruch 5 aufweisen.

**10.** Zur Bekämpfung von pflanzenpathogenen Schadpilzen geeignetes Mittel, enthaltend einen festen oder flüssigen Trägerstoff und eine Verbindung der Formel I gemäß Ansprüchen 1 bis 5.

**11.** Verfahren zur Bekämpfung von pflanzenpathogenen Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der Formel I gemäß Ansprüchen 1 bis 5 behandelt.

## Claims

**1.** A 2-(2-pyridyl)-5-phenyl-6-aminopyrimidi.ne of the formula I,

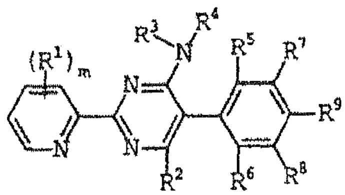

I

in which the substituents and the subscript have the following meanings:

$R^1$ is halogen, hydroxyl, cyano, oxo, nitro, amino, mercapto, $C_1$-$C_6$-alkyl, $C_1$-$C_6$-haloalkyl, $C_2$-$C_6$-alkenyl, $C_2$-$C_6$-alkynyl, $C_3$-$C_6$-cycloalkyl, $C_1$-$C_6$-alkoxy, $C_1$-$C_6$-haloalkoxy, carboxyl, $C_1$-$C_7$-alkoxycarbonyl, carbamoyl, $C_1$-$C_7$-alkylaminocarbonyl, $C_1$-$C_6$-alkyl-$C_1$-$C_6$-alkylaminocarbonyl, morpholinocarbonyl, pyrrolidinocarbonyl, $C_1$-$C_7$-alkylcarbonylamino, $C_1$-$C_6$-alkylamino, di($C_1$-$C_6$-alkyl) amino, $C_1$-$C_6$-alkylthio, $C_1$-$C_6$-alkylsulfinyl, $C_1$-$C_6$-alkylsulfonyl, hydroxysulfonyl, aminosulfonyl, $C_1$-$C_6$-alkylaminosulfonyl or di($C_1$-$C_6$-alkyl)aminosulfonyl;
m is 0, 1, 2, 3 or 4;
$R^2$ is halogen, cyano, $C_1$-$C_6$-alkyl, $C_1$-$C_6$-haloalkyl, $C_1$-$C_6$-alkoxy, $C_1$-$C_6$-haloalkoxy or $C_3$-$C_6$-alkenyloxy;
$R^3$, $R^4$ independently of one another, are hydrogen, $C_1$-$C_6$-alkyl, $C_1$-$C_6$-haloalkyl, $C_3$-$C_6$-cycloalkyl, $C_3$-$C_6$-halocycloalkyl, $C_2$-$C_6$-alkenyl, $C_2$-$C_6$-haloalkenyl, $C_3$-$C_6$-cycloalkenyl, $C_2$-$C_6$-alkynyl, $C_2$-$C_6$-haloalkynyl or $C_3$-$C_6$-cycloalkynyl,
$R^3$ and $R^4$ can also, together with the nitrogen atom to which they are bonded, form a five- or six-membered ring which may be interrupted by an atom from the group consisting of O, N and S and/or may carry one or more substituents from the group consisting of halogen, $C_1$-$C_6$-alkyl, $C_1$-$C_6$-haloalkyl or oxy-$C_1$-$C_3$-alkylenoxy or in which two adjacent carbon atoms or one N- and one neighboring carbon atom can be connected via a $C_1$-$C_4$-alkylene chain;
$R^5$ is halogen, $C_1$-$C_6$-alkyl or $C_1$-$C_6$-haloalkyl;
$R^6$ is hydrogen or one of the groups mentioned under $R^5$;
$R^7$, $R^8$ independently of one another, are hydrogen, halogen, $C_1$-$C_6$-alkyl or $C_1$-$C_6$-haloalkyl;
$R^9$ is hydrogen, halogen, hydroxyl, cyano, $C_1$-$C_6$-alkyl, $C_1$-$C_6$-alkoxy, $C_3$-$C_6$-cycloalkoxy, $C_1$-$C_6$-haloalkoxy, $C_1$-$C_6$-alkoxycarbonyl or $C_1$-$C_6$-alkylaminocarbonyl.

**2.** The compound of the formula I according to claim 1, wherein m is zero or 1, 2 or 3 and $R^1$ has the following meaning:

halogen, hydroxyl, cyano, nitro, amino, mercapto, $C_1$-$C_6$-alkyl, $C_1$-$C_6$-haloalkyl, $C_2$-$C_6$-alkenyl, $C_2$-$C_6$-alkynyl, $C_3$-$C_6$-cycloalkyl, $C_1$-$C_6$-alkoxy, $C_1$-$C_6$-haloalkoxy, carboxyl, $C_1$-$C_7$-alkoxycarbonyl, carbamoyl, $C_1$-$C_7$-alkylaminocarbonyl, $C_1$-$C_6$-alkyl-$C_1$-$C_6$-alkylaminocarbonyl, morpholinocarbonyl, pyrrolidinocarbonyl, $C_1$-$C_7$-alkylcarbonylamino, $C_1$-$C_6$-alkylamino, di($C_1$-$C_6$-alkyl) amino, $C_1$-$C_6$-alkylthio, $C_1$-$C_6$-alkylsulfinyl, $C_1$-$C_6$-alkylsulfonyl, hydroxysulfonyl, aminosulfonyl, $C_1$-$C_6$-alkylaminosulfonyl or di($C_1$-$C_6$-alkyl)aminosulfonyl.

**3.** The compound of the formula I according to claim 2, wherein the variables have the following meanings:

$R^2$ is halogen, $C_1$-$C_6$-alkyl or $C_1$-$C_6$-alkoxy;
$R^3$, $R^4$ independently of one another, are hydrogen, $C_1$-$C_6$-alkyl, $C_1$-$C_6$-haloalkyl, $C_3$-$C_6$-cycloalkyl or $C_2$-$C_6$-alkenyl;
$R^3$ and $R^4$ can also, together with the nitrogen atom to which they are bonded, form a five- or six-membered ring which may be interrupted by an oxygen atom or may carry a $C_1$-$C_6$-alkyl substituent;
$R^5$, $R^6$ independently of one another, are halogen;
$R^7$, $R^8$ independently of one another, are halogen;
$R^9$ is hydrogen, halogen, hydroxyl, $C_1$-$C_6$-alkoxy or $C_1$-$C_6$-alkoxycarbonyl.

**4.** The compound of the formula I according to any of claims 1 to 3, wherein $R^2$ represents chlorine.

**5.** The compound of the formula I according to any of claims 1 to 4, wherein the combination of the substituents $R^5$ to $R^9$ has the following meanings: 2-methyl-4-fluoro; 2-fluoro-4-methyl; 2,4-dimethyl; 2-chloro-6-fluoro; 2,6-difluoro; 2,6-dichloro; 2-methyl-6-fluoro; 2,4,6-trifluoro; 2,6-difluoro-4-methoxy and pentafluoro.

**6.** A process for the preparation of a 5-phenylpyrimidine of the formula I according to any of claims 1 to 5 in which $R^2$

is chlorine, which comprises reacting a 2-pyridylamidine of the formula II,

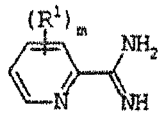

II

with a phenylmalonate of the formula III,

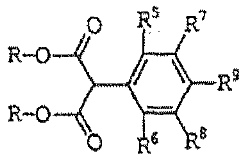

III

in which R is $C_1$-$C_6$-alkyl, to give a compound of the formula IV,

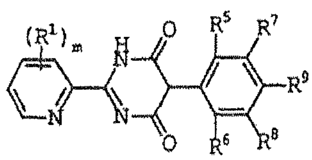

IV

which is converted by a chlorinating agent to a dichloropyrimidine of the formula V

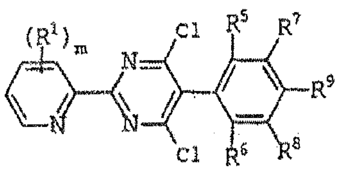

V

which is converted, with an amine of the formula VI

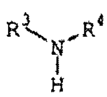

VI

to a pyrimidine derivative of the formula I in which $R^2$ is chlorine.

**7.** A process for the preparation of a 5-phenylpyrimidine of the formula I according to any of claims 1 to 5 in which $R^2$ is $C_1$-$C_6$-alkyl or $C_1$-$C_6$-haloalkyl, which comprises reacting a 2-pyridylamidine of the formula II according to claim 6 with a phenyl-β-ketoester of the formula VII,

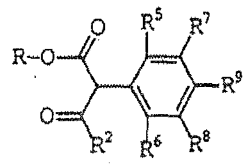

VII

in which R is $C_1$-$C_6$-alkyl, to give a compound of the formula IVa

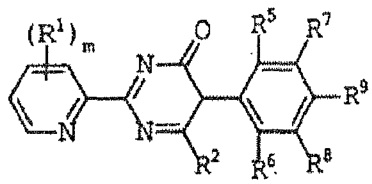

IVa

which is converted by a chlorinating agent to a chloropyrimidine of the formula Va

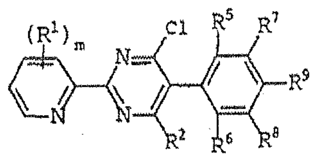

Va

which is converted, with an amine VI according to claim 6, to a pyrimidine derivative of the formula I in which $R^2$ is $C_1$-$C_6$-alkyl or $C_1$-$C_6$-haloalkyl.

**8.** An intermediate of the formula IV or V according to claim 6, wherein the combination of the substituents $R^5$ to $R^9$ has the meanings according to claim 5.

**9.** An intermediate of the formula IVa or Va according to claim 7, wherein the combination of the substituents $R^5$ to $R^9$ has the meanings according to claim 5.

**10.** A composition suitable for the control of harmful phytopathogenic fungi, comprising a solid or liquid carrier and a compound of the formula I according to any of claims 1 to 5.

**11.** A method for the control of harmful phytopathogenic fungi, which comprises treating the fungi or the materials, plants, ground or seeds to be protected from fungal attack with an effective amount of a compound of the formula I according to any of claims 1 to 5.

**Revendications**

**1.** 2-(2-Pyridyl)-5-phényl-6-aminopyrimidines de formule I,

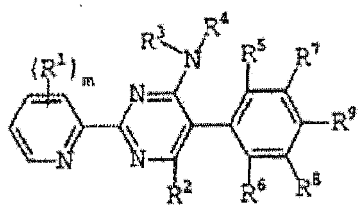

I

dans laquelle les substituants et l'indice ont la signification suivante :

$R^1$ halogène, hydroxy, cyano, oxo, nitro, amino, mercapto, alkyle en $C_1$-$C_6$, halogénoalkyle en $C_1$-$C_6$, alcényle en $C_2$-$C_6$, alcynyle en $C_2$-$C_6$, cycloalkyle en $C_3$-$C_6$, alcoxy en $C_1$-$C_6$, halogénoalcoxy en $C_1$-$C_6$, carboxyle, alcoxycarbonyle en $C_1$-$C_7$, carbamoyle, alkylaminocarbonyle en $C_1$-$C_7$, $C_1$-$C_6$-alkyl-$C_1$-$C_6$-alkylaminocarbonyle, morpholinocarbonyle, pyrrolidinocarbonyle, alkylcarbonylamino en $C_1$-$C_7$, alkylamino en $C_1$-$C_6$, di-($C_1$-$C_6$-alkyl)amino, alkylthio en $C_1$-$C_6$, alkylsulfinyle en $C_1$-$C_6$, alkylsulfonyle en $C_1$-$C_6$, hydroxysulfonyle, aminosulfonyle, alkylaminosulfonyle en $C_1$-$C_6$, di-($C_1$-$C_6$-alkyle)aminosulfonyle ;
m 0, 1, 2, 3 ou 4 ;
$R^2$ halogène, cyano, alkyle en $C_1$-$C_6$, halogénoalkyle en $C_1$-$C_6$, alcoxy en $C_1$-$C_6$, halogénoalcoxy en $C_1$-$C_6$ ou alcényloxy en $C_3$-$C_6$ ;
$R^3$, $R^4$ indépendamment l'un de l'autre, hydrogène, alkyle en $C_1$-$C_6$, halogénoalkyle en $C_1$-$C_6$, cycloalkyle en $C_3$-$C_6$, halogénocycloalkyle en $C_3$-$C_6$, alcényle en $C_2$-$C_6$, halogénoalcényle en $C_2$-$C_6$, cycloalcényle en $C_3$-$C_6$, alcynyle en $C_2$-$C_6$, halogénoalcynyle en $C_2$-$C_6$ ou cycloalcynyle en $C_3$-$C_6$,
$R^3$ et $R^4$ pouvant également former avec l'atome d'azote auquel ils sont reliés un cycle à cinq ou six éléments, qui peut être interrompu par un atome du groupe constitué par O, N ou S et/ou porter un ou plusieurs substituants du groupe constitué par halogène, alkyle en $C_1$-$C_6$, halogénoalkyle en $C_1$-$C_6$ ou oxy-$C_1$-$C_3$-alkylènoxy ou dans lequel deux atomes C adjacents ou un atome N et un atome C adjacents peuvent être reliés par une chaîne alkylène en $C_1$-$C_4$ ;
$R^5$ halogène, alkyle en $C_1$-$C_6$ ou halogénoalkyle en $C_1$-$C_6$ ;
$R^6$ hydrogène ou des groupes cités pour $R^5$ ;
$R^7$, $R^8$ indépendamment l'un de l'autre, hydrogène, halogène, alkyle en $C_1$-$C_6$ ou halogénoalkyle en $C_1$-$C_6$ ;
$R^9$ hydrogène, halogène, hydroxy, cyano, alkyle en $C_1$-$C_6$, alcoxy en $C_1$-$C_6$, cycloalcoxy en $C_3$-$C_6$, halogénoalcoxy en $C_1$-$C_6$, alcoxycarbonyle en $C_1$-$C_6$ ou alkylaminocarbonyle en $C_1$-$C_6$.

**2.** Composés de formule I selon la revendication 1, dans lesquels m représente zéro, 1, 2 ou 3 et $R^1$ a la signification suivante :

halogène, hydroxy, cyano, nitro, amino, mercapto, alkyle en $C_1$-$C_6$, halogénoalkyle en $C_1$-$C_6$, alcényle en $C_2$-$C_6$, alcynyle en $C_2$-$C_6$, cycloalkyle en $C_3$-$C_6$, alcoxy en $C_1$-$C_6$, halogénoalcoxy en $C_1$-$C_6$, carboxyle, alcoxycarbonyle en $C_1$-$C_7$, carbamoyle, alkylaminocarbonyle en $C_1$-$C_7$, $C_1$-$C_6$-alkyl-$C_1$-$C_6$-alkylaminocarbonyle, morpholinocarbonyle, pyrrolidinocarbonyle, alkylcarbonylamino en $C_1$-$C_7$, alkylamino en $C_1$-$C_6$, di-($C_1$-$C_6$-alkyl)amino, alkylthio en $C_1$-$C_6$, alkylsulfinyle en $C_1$-$C_6$, alkylsulfonyle en $C_1$-$C_6$, hydroxysulfonyle, aminosulfonyle, alkylaminosulfonyle en $C_1$-$C_6$, di-($C_1$-$C_6$-alkyle)aminosulfonyle.

**3.** Composés de formule I selon la revendication 2, dans lesquels les variables ont la signification suivante :

$R^2$ halogène, alkyle en $C_1$-$C_6$ ou alcoxy en $C_2$-$C_6$ ;
$R^3$, $R^4$ indépendamment l'un de l'autre, hydrogène, alkyle en $C_1$-$C_6$, halogénoalkyle en $C_1$-$C_6$, cycloalkyle en $C_3$-$C_6$ ou alcényle en $C_2$-$C_6$
$R^3$ et $R^4$ pouvant également former avec l'atome d'azote auquel ils sont reliés un cycle à cinq ou six éléments, qui peut être interrompu par un atome d'oxygène et porter un substituant alkyle en $C_1$-$C_6$ ;
$R^5$, $R^6$ indépendamment l'un de l'autre, halogène ;
$R^7$, $R^8$ indépendamment l'un de l'autre, hydrogène ou halogène ;
$R^9$ hydrogène, halogène, hydroxy, alcoxy en $C_1$-$C_6$ ou alcoxycarbonyle en $C_1$-$C_6$.

**4.** Composés de formule I selon les revendications 1 à 3, dans lesquels $R^2$ représente le chlore.

**5.** Composés de formule I selon les revendications 1 à 4, dans lesquels la combinaison des substituants $R^5$ à $R^9$ a les significations suivantes : 2-méthyl,4-fluoro ; 2-fluoro,4-méthyl ; 2,4-diméthyl ; 2-chloro,6-fluoro ; 2,6-difluoro ; 2,6-dichloro ; 2-méthyl,6-fluoro ; 2,4,6-trifluoro ; 2,6-difluoro ; 4-méthoxy et pentafluoro.

**6.** Procédé de fabrication de 5-phénylpyrimidines de formule I selon les revendications 1 à 5, dans laquelle $R^2$ représente le chlore, **caractérisé en ce que** des 2-pyridylamidines de formule II,

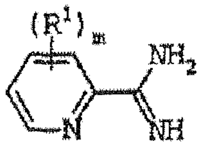

II

sont mises en réaction avec des phénylmalonates de formule III,

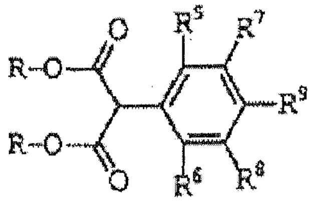

III

dans laquelle R représente un alkyle en $C_1$-$C_6$, pour former des composés de formule IV,

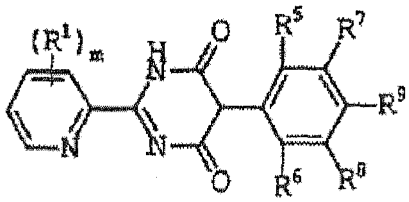

IV

qui sont convertis avec des agents de chloration en dichloropyrimidines de formule V

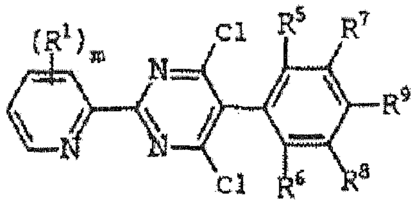

V

qui sont mises en réaction avec des amines de formule VI

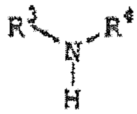

VI

pour former les dérivés de pyrimidine de formule I, dans lesquels $R^2$ représente le chlore.

**7.** Procédé de fabrication de 5-phénylpyrimidines de formule I selon les revendications 1 à 5, dans laquelle $R^2$ représente un alkyle en $C_1$-$C_6$ ou un halogénoalkyle en $C_1$-$C_6$, **caractérisé en ce qu'**une 2-pyridylamidine de formule II selon la revendication 6 est mise en réaction avec des phényl-β-cétoesters de formule VII,

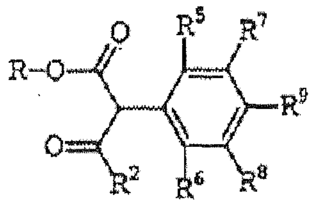

VII

dans laquelle R représente un alkyle en $C_1$-$C_6$, pour former des composés de formule IVa

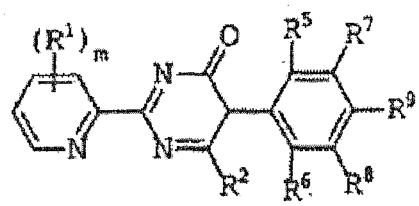

IVa

qui sont convertis avec des agents de chloration en chloropyrimidines de formule Va

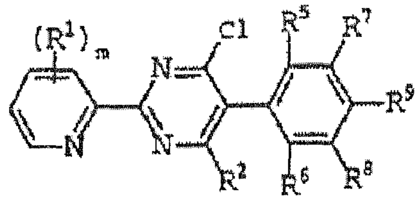

Va

qui sont mises en réaction avec des amines VI selon la revendication 6 pour former les dérivés de pyrimidine de formule I, dans laquelle $R^2$ représente un alkyle en $C_1$-$C_6$ ou un halogénoalkyle en $C_1$-$C_6$.

**8.** Produits intermédiaires de formules IV et V selon la revendication 6, dans lesquels la combinaison des substituants $R^5$ à $R^9$ a les significations données dans la revendication 5.

**9.** Produits intermédiaires de formules IVa et Va selon la revendication 7, dans lesquels la combinaison des substituants $R^5$ à $R^9$ a les significations données dans la revendication 5.

**10.** Agent approprié pour la lutte contre les champignons phytopathogènes, contenant un véhicule solide ou liquide et un composé de formule I selon les revendications 1 à 5.

**11.** Procédé de lutte contre les champignons phytopathogènes, **caractérisé en ce que** les champignons ou les matériaux, les plantes, les sols ou les semences à protéger avant l'infestation des champignons sont traités avec une quantité efficace d'un composé de formule I selon les revendications 1 à 5.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 407899 A
- EP 588146 A
- EP 1002788 A
- WO 9841496 A
- US 4963678 A
- EP 745593 A
- DE 19642533 A
- WO 9932458 A


### In der Beschreibung aufgeführte Nicht-Patentliteratur


- *Austr. J. Chem.,* 1979, vol. 32, 669-679
- *J. Org. Chem.,* 1993, vol. 58, 3785-3786
- *Arm. Xim. ZH,* 1985, vol. 38 (11), 718-719
- *J.Org. Chem.,* 1993, vol. 58, 3785-3786
- *Helv. Chim. Acta,* 1981, vol. 64, 113-152
- *J. Chem. Res.,* 1995, vol. S (7), 286-287
- *Liebigs Ann. Chem.,* 1995, 1703-1705
- *Heterocycles,* 1991, vol. 32, 1327-1340
- *J. Heterocycl. Chem.,* 1982, vol. 19, 1565-1567
- *Geterotsikl. Soedin,* 1991, 400-402
- *Heterocycles,* 1994, vol. 39, 345-356
- *Collect. Czech. Chem. Commun.,* 1995, vol. 60, 1386-1389
- *Acta Chim. Scand.,* 1996, vol. 50, 58-63